# EUROPÄISCHE PATENTSCHRIFT

(11) **EP 0 882 043 B1**
(45) Veröffentlichungstag und Bekanntmachung des Hinweises auf die Patenterteilung: **04.09.2002**
(21) Anmeldenummer: 97900603.8
(22) Anmeldetag: 15.01.1997
(51) Int. Cl.: C07D 413/12, C07D 273/00, C07D 413/14, A01N 43/88

(54) **HALOGENPYRIMIDINE UND IHRE VERWENDUNG ALS SCHÄDLINGSBEKÄMPFUNGSMITTEL**
HALOGEN PYRIMIDINES AND ITS USE THEREOF AS PARASITE ABATEMENT MEANS
PYRIMIDINES HALOGENEES ET LEUR UTILISATION EN TANT QU'AGENTS DE LUTTE CONTRE LES PARASITES

(30) Priorität: 22.01.1996 DE 19602095
(43) Veröffentlichungstag der Anmeldung: 09.12.1998
(73) Patentinhaber: BAYER AG, 51368 Leverkusen (DE)
(72) Erfinder: HEINEMANN, Ulrich, D-42799 Leichlingen (DE); GAYER, Herbert, D-40789 Monheim (DE); GERDES, Peter, D-52080 Aachen (DE); KRÜGER, Bernd-Wieland, D-51467 Bergisch Gladbach (DE); GALLENKAMP, Bernd, D-42113 Wuppertal (DE); STELZER, Uwe, D-51399 Burscheid (DE); MARHOLD, Albrecht, D-51373 Leverkusen (DE); TIEMANN, Ralf, D-51375 Leverkusen (DE); DUTZMANN, Stefan, D-40764 Langenfeld (DE); HÄNSSLER, Gerd, D-51381 Leverkusen (DE); STENZEL, Klaus, D-40595 Düsseldorf (DE)
(86) Internationale Anmeldenummer: EP9700151
(87) Internationale Veröffentlichungsnummer: WO97027189

(56) Entgegenhaltungen:
- DE-A- 4 408 005
- DE-A- 19 501 842

## Beschreibung

Die Erfindung betrifft neue Halogenpyrimidine, ein Verfahren zu ihrer Herstellung und ihre Verwendung als Schädlingsbekämpfungsmittel.

Bestimmte Pyrimidine mit ähnlichem Substitutionsmuster sind bereits bekannt geworden (WO-A 9504728).

Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Halogenpyrimidine der allgemeinen Formel (I) gefunden, 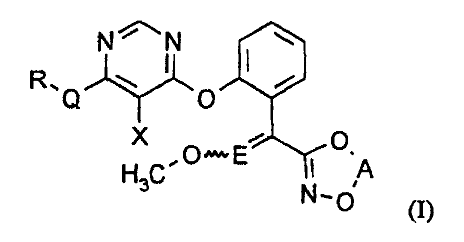 in welcher
- A: für gegebenenfalls durch Halogen substituiertes Alkandiyl mit 1 bis 5 Kohlenstoffatomen steht,
- R: für jeweils gegebenenfalls einfach bis zweifach durch Halogen, Alkyl, oder Hydroxy substiuiertes Cycloalkyl mit 3 bis 7 Kohlenstoffatomen;
für gegebenenfalls durch Alkyl mit 1 bis 4 Kohlenstoffatomen substituiertes Benzodioxanyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 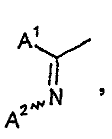 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl, Phenylalkyl, Phenylalkyloxy, Phenylalkylthio oder Heterocyclylalkyl mit jeweils 1 bis 3 Kohlenstoffatomen in den jeweiligen Alkylteilen,
- E: für -CH= oder Stickstoff steht,
- Q: für Sauerstoff, Schwefel, eine Einfachbindung oder für ein gegebenenfalls durch Methyl, Ethyl oder n- oder i-Propyl substituiertes Stickstoffatom steht und
- X: für Fluor, Chlor, Brom oder Iod steht.

Werterhin wurde gefunden, daß man die neuer Halogenpyrimidine der allgemeinen Formel (I) erhält, wenn man
a) Hydroxyverbindungen der allgemeinen Formel (II) 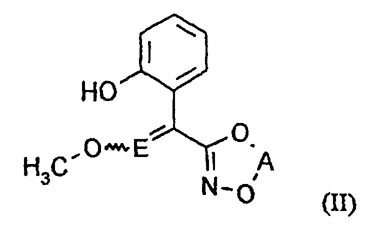 in welcher
   - A und E: die oben angegebenen Bedeutungen haben,
   mit einem substituierten Halogenpyrimidin der allgemeinen Formel (III), 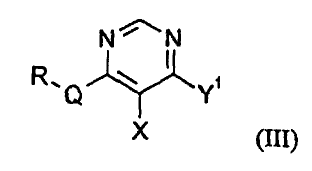 in welcher
   - R, Q und X: die oben angegebenen Bedeutungen haben und
   - Y¹: für Halogen steht,
   gegebenenfalls in Gegenwart eines Verdünnungsmittels, gegebenenfalls in Gegenwart eines Säureakzeptors und gegebenenfalls in Gegenwart eines Katalysators, umsetzt, oder wenn man
b) Phenoxypyrimidine der allgemeinen Formel (IV) 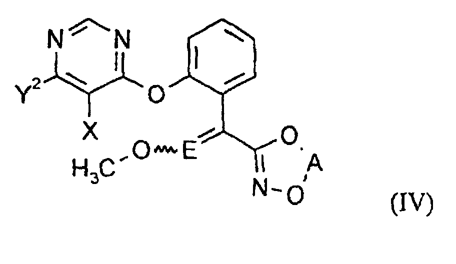
in welcher
- A, E und X: die oben angegebenen Bedeutungen haben und
- Y²: für Halogen steht,
mit einer Ringverbindung der allgemeinen Formel (V),

R―Q―H (V)

in welcher
- R und Q: die oben angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators, umsetzt.

Schließlich wurde gefunden, daß die neuen Halogenpyrimidine der allgemeinen Formel (I) sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt.

Die Erfindung betrifft insbesondere Verbindungen der Formel (I), in welcher
- A: für jeweils gegebenenfalls durch Fluor oder Chlor substituiertes Methylen, Ethan-1, 1-diyl, Ethan-1,2-diyl, Propan-1,1-diyl, Propan-1,2-diyl, Propan-1,3-diyl, Butan-1,1-diyl, Butan-1,2-diyl, Butan-1,3-diyl oder Butan-2,2-diyl, steht
- R: für jeweils gegebenenfalls einfach bis zweifach durch Fluor, Chlor, Methyl, Ethyl oder Hydroxy substiuiertes Cyclopentyl oder Cyclohexyl;
für gegebenenfalls durch Methyl oder Ethyl substituiertes Benzodioxanyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 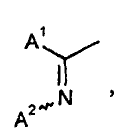 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
- E: für -CH= oder Stickstoff steht,
- Q: für Sauerstoff, Schwefel, eine Einfachbindung oder für ein gegebenenfalls durch Methyl substituiertes Stickstoffatom steht und
- X: für Fluor oder Chlor steht.

Die oben aufgeführten allgemeinen oder in Vorzugsbereichen angegebenen Restedefinitionen gelten sowohl fiir die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Die zur Durchführung des erfindungsgemäßen Verfahrens a) als Ausgangsstoffe benötigten Hydroxyverbindungen sind durch die Formel (II) allgemein definiert. In dieser Formel (II) haben A und E vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und E angegeben wurden.

Die Ausgangsstoffe der Formel (II) sind teilweise bekannt und/oder können nach an sich bekannten Verfahren hergestellt werden (vgl. WO-A 9504728). Neu, und auch Gegenstand der vorliegenden Anmeldung, sind Methoxyvinylverbindungen der allgemeinen Formel (IIa), 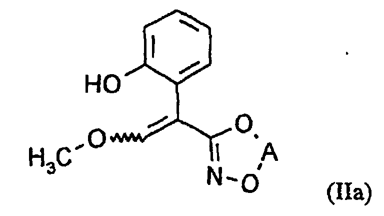 in welcher
- A: die oben angegebene Bedeutung hat.

Die Methoxyvinylverbindungen der Formel (IIa) werden erhalten, wenn man (Verfahren a-1) Tetrahydropyranylether der allgemeinen Formel (VI), 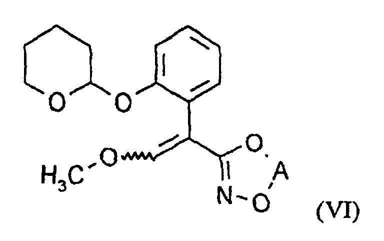 in welcher
- A: die oben angegebene Bedeutung hat,
gegebenenfalls in Gegenwart eines Verdünnungsmittels, vorzugsweise eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser
mit einer Säure, vorzugsweise einer anorganischen oder organischen Protonen- oder Lewissäure, wie beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, oder auch einer polymeren Säure wie beispielsweise einem sauren Ionenaustauscher, einer sauren Tonerde oder saurem Kieselgel,
bei Temperaturen von -20°C bis 120°C, vorzugsweise bei Temperaturen von -10°C bis 80°C,
behandelt.

Die zur Durchführung des erfindungsgemäßen Verfahrens a-1) als Ausgangsstoffe benötigten Tetrahydropyranylether sind durch die Formel (VI) allgemein definiert. In dieser Formel (VI) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde.

Die Ausgangsstoffe der Formel (VI) sind neu und auch Gegenstand der vorliegenden Anmeldung.

Die Tetrahydropyranylether der Formel (VI) werden erhalten, wenn man (Verfahren a-2) Ketoverbindungen der allgemeinen Formel (VII), 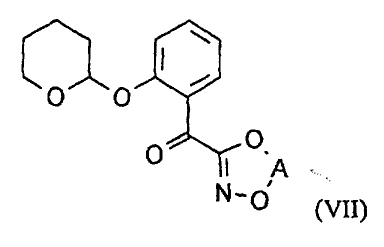 in welcher
- A: die oben angegebene Bedeutung hat,
mit Methoxymethyl-triphenyl-phosphonium-chlorid, -bromid oder -iodid, gegebenenfalls in Gegenwart eines Verdünnungsmittels, vorzugsweise eines inerten, aprotischen Lösungsmittels, wie beispielsweise eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Amids, wie N,N-Dimethylformamid, N,N-Dimethylacetamid, N-Methylformanilid, N-Methylpyrrolidon oder Hexamethylphosphorsäuretriamid; eines Sulfoxids, wie Dimethylsulfoxid, oder eines Sulfons, wie Sulfolan, und
gegebenenfalls in Gegenwart einer Base, vorzugsweise eines Erdalkalimetall- oder Alkalimetallhydrids, -hydroxids, -amids oder -alkoholates, wie beispielsweise Natriumhydrid, Natriumamid, Natrium-methylat, Natrium-ethylat, Kalium-tert.-butylat, Natriumhydroxid oder Kaliumhydroxid,
bei Temperaturen von 0°C bis 100°C, vorzugsweise von 20°C bis 80°C, umsetzt.

Die zur Durchführung des erfindungsgemäßen Verfahrens a-2) als Ausgangsstoffe benötigten Ketoverbindungen sind durch die Formel (VII) allgemein definiert. In dieser Formel (VII) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde.

Die Ausgangsstoffe der Formel (VII) sind neu und auch Gegenstand der vorliegenden Anmeldung.

Die Ketoverbindungen der Formel (VII) werden erhalten, wenn man (Verfahren a-3) Halogenphenylverbindungen der allgemeinen Formel (VIII), 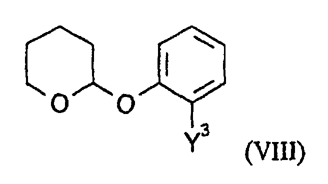 in welcher
- Y³: für Halogen steht,
mit Amiden der Formel (IX), 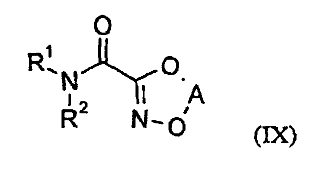 in welcher
- A: die oben angegebene Bedeutung hat und
- R¹ und R²: gleich oder verschieden sind und für Alkyl stehen, oder gemeinsam mit dem Stickstoffatom, an das sie gebunden sind, für einen 3 bis 8-gliedrigen, gesättigten, heterocyclischen Ring stehen,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin, oder eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol und
gegebenenfalls in Gegenwart einer Base, vorzugsweise eines Erdalkalimetall- oder Alkalimetallhydrids, -amids, wie beispielsweise Natriumhydrid oder Natriumamid, oder einer Alkali- oder Erdalkalimetall-Kohlenwasserstoffverbindung, wie Butyllithium,
bei Temperaturen von -80 bis 20°C, vorzugsweise -60 bis -20°C, umsetzt.

Die zur Durchführung des erfindungsgemäßen Verfahrens a-3) als Ausgangsstoffe benötigten Halogenphenylverbindungen sind durch die Formel (VIII) allgemein definiert. In dieser Formel (VIII) steht Y³ für Halogen, bevorzugt für Brom.

Die Ausgangsstoffe der Formel (VIII) sind bekannt und/oder können nach bekannten Methoden hergestellt werden (vergleiche z. B. Synthesis 1987, 951).

Die zur Durchführung des erfindungsgemäßen Verfahrens a-3) weiterhin als Ausgangsstoffe benötigten Amide sind durch die Formel (IX) allgemein definiert. In dieser Formel (IX) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde. R¹ und R² sind gleich oder verschieden und stehen für Alkyl, vorzugsweise für Methyl, Ethyl, n- oder i-Propyl oder n-, i-, s- oder t-Butyl, oder stehen gemeinsam mit dem Stickstoffatom, an das sie gebunden sind, für einen 3 bis 8-gliedrigen, gesättigten, heterocyclischen Ring, vorzugsweise für Azetidinyl, Pyrrolidinyl, Morpholinyl, Piperidinyl, Hexahydroazepinyl.

Die Ausgangsstoffe der Formel (IX) sind neu und auch Gegenstand der vorliegenden Anmeldung.

Die Amide der Formel (IX) werden erhalten, wenn man (Verfahren a-4) Oxalsäureesteramide der allgemeinen Formel (X), 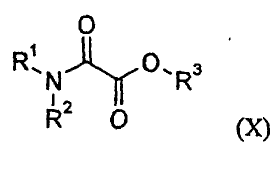 in welcher
- R¹ und R²: die oben angegebenen Bedeutungen haben und
- R³: für Alkyl steht,
zunächst mit Hydroxylamin oder einem seiner Säureadditionssalze, gegebenenfalls in Gegenwart eines Verdünnungsmittels, vorzugsweise eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether,
und gegebenenfalls in Gegenwart einer Base, vorzugsweise eines Erdalkalimetallhydroxids, -alkoholates, -acetates, -carbonates oder -hydrogencarbonates, wie beispielsweise Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat,
bei Temperaturen von -20 bis 50°C, vorzugsweise 0 bis 40°C, umsetzt,
und die so erhaltene Hydroxamsäure der Formel (XI) 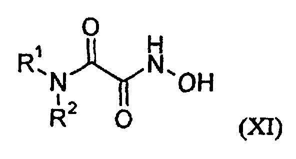 ohne Aufarbeitung mit einem Alkylenderivat der allgemeinen Formel (XII),

Y⁴―A―Y⁵ (XII)

in welcher
- A: die oben angegebene Bedeutung hat und
- Y⁴ und Y⁵: gleich oder verschieden sind und für Halogen, Alkylsulfonyl oder Arylsulfonyl stehen,
gegebenenfalls in Gegenwart eines Verdünnungsmittels, vorzugsweise eines Alkoholes und gegebenenfalls in Gegenwart einer Base, vorzugsweise eines Erdalkalimetall-hydroxids, -alkoholates, -acetates, -carbonates oder -hydrogencarbonates, wie beispielsweise Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, umsetzt.

Die zur Durchführung der ersten Stufe des erfindungsgemäßen Verfahrens a-4) als Ausgangsstoffe benötigten Oxalsäureesteramide sind durch die Formel (X) allgemein definiert. In dieser Formel (X) haben R¹ und R² vorzugsweise bzw. insbesondere diejenigen Bedeutungen, die bereits im Zusammenhang mit der Beschreibung der erfindungsgemäßen Verbindungen der Formel (IX) als bevorzugt bzw. als insbesondere bevorzugt für R¹ und R² angegeben wurden. R³ steht für Alkyl, vorzugsweise für Methyl oder Ethyl.

Die Ausgangsstoffe der Formel (X) sind bekannt und/oder können nach bekannten Methoden hergestellt werden (vergleiche z. B. EP-A 469889).

Das weiterhin zur Durchführung der ersten Stufe des erfindungsgemäßen Verfahrens a-4) benötigte Hydroxylamin oder dessen Salze sind allgemein übliche Synthesechemikalien.

Die zur Durchführung der zweiten Stufe des erfindungsgemäßen Verfahrens a-4) als Ausgangsstoffe benötigten Alkylenderivate sind durch die Formel (XII) allgemein definiert. In dieser Formel (XII) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde. Y⁴ und Y⁵ sind gleich oder verschieden und stehen für Halogen, vorzugsweise für Chlor oder Brom; Alkylsulfonyl, vorzugsweise Methansulfonyl; oder Arylsulfonyl, vorzugsweise Toluolsulfonyl.

Die zur Durchführung des erfindungsgemäßen Verfahrens a) weiterhin als Ausgangsstoffe benötigten Halogenpyrimidine sind durch die Formel (III) allgemein definiert. In dieser Formel (III) haben R, Q und X vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R, Q und X angegeben wurden, Y¹ steht für Halogen, bevorzugt für Fluor oder Chlor.

Die Ausgangsstoffe der Formel (III) sind bekannt und/oder können nach bekannten Methoden hergestellt werden (vergleiche z. B. DE-A 4340181; Chem.Ber., 90 <1957> 942, 951).

Die zur Durchführung des erfindungsgemäßen Verfahrens b) als Ausgangsstoffe benötigten Phenoxypyrimidine sind durch die Formel (IV) allgemein definiert. In dieser Formel (IV) haben A, E und X vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A, E und X angegeben wurden. Y² steht für Halogen, vorzugsweise für Fluor oder Chlor.

Die Ausgangsstoffe der Formel (IV) sind neu und ebenfalls Gegenstand der vorliegenden Anmeldung.

Die Phenoxypyrimidine der allgemeinen Formel (IV) werden erhalten (Verfahren b-1), wenn man Hydroxyverbindungen der allgemeinen Formel (II) mit einem Trihalogenpyrimidin der allgemeinen Formel (XIII) 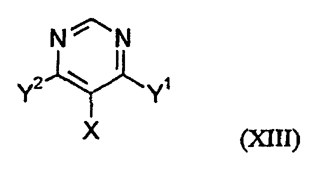 in welcher
- X, Y¹ und Y²: gleich oder verschieden sind und jeweils für Halogen stehen,
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens b-1) als Ausgangsstoffe benötigten Hydroxyverbindungen der Formel (II) sind bereits im Zusammenhang mit der Beschreibung des erfindungsgemäßen Verfahrens a) beschrieben worden.

Die weiterhin zur Durchführung des erfindungsgemäßen Verfahrens b-1) als Ausgangsstoffe benötigten Trihalogenpyrimidine sind durch die Formel (XIII) allgemein definiert. In dieser Formel (XIII) stehen X, Y¹ und Y² für Halogen, vorzugsweise für Fluor oder Chlor.

Die Trihalogenpyrimidine sind bekannt und/oder können nach bekannten Methoden hergestellt werden (vergleiche z. B. Chesterfield et al., J. Chem. Soc., 1955; 3478, 3480).

Die weiterhin zur Durchführung des erfindungsgemäßen Verfahrens b) als Ausgangsstoffe benötigten Ringverbindungen sind durch die Formel (V) allgemein definiert. In dieser Formel (V) haben R und Q vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R und Q angegeben wurden.

Die Ringverbindungen der Formel (V) sind bekannte Synthesechemikalien oder können nach einfachen Methoden hergestellt werden.

Als Verdünnungsmittel zur Durchführung der erfindungsgemäßen Verfahren a), b) und b-1) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Die erfindungsgemäßen Verfahren a), b) und b-1) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -alkoholate, -carbonate oder -hydrogencarbonate, wie beispielsweise Natriumhydrid, Natriumamid, Kalium-tert.butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren a), b) und b-1) eignen sich alle Kupfer(I)-Salze, wie beispielsweise Kupfer(I)-chlorid, Kupfer(I)-bromid oder Kupfer(I)-iodid.

Die Reaktionstemperaturen können bei der Durchführung der erfindungsgemäßen Verfahren a), b) und b-1) in einem größeren Bereich variiert werden. Im allgemeinen arbeitet man bei Temperaturen von -20°C bis 100°C, vorzugsweise bei Temperaturen von -10°C bis 80°C.

Alle erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach allgemein üblichen Verfahren (vergleiche auch die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Getreidekrankheiten, wie beispielsweise gegen Erysiphe-, Fusarium-, Pseudocercosporella- und Puccinia-Arten oder von Krankheiten im Wein-, Obst- und Gemüseanbau, wie beispielsweise gegen Venturia-, Sphaerotheca-, Phytophthora- und Plasmopara-Arten, oder auch von Reiskrankheiten, wie beispielsweise Pyricularia-Arten, eingesetzt. Die erfindungsgemäßen Wirkstoffe weisen ferner eine sehr starke und breite in vitro-Wirkung auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexformige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(otolyloxy)-o-]yl]-acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, Chloethocarb, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin,
   Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1

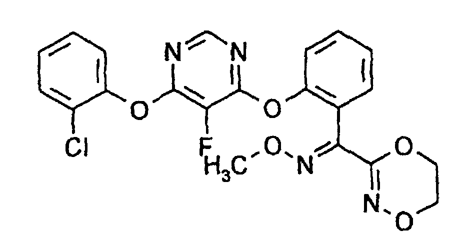

### Verfahren a)

Zu einer Mischung aus 135,3 g ( 0,56 Mol) 3-[1-(2-Hydroxyphenyl)-1-(methoximino)-methyl]-5,6-dihydro-1,4,2-dioxazin und 197,6 g gemahlenem Kaliumcarbonat in 460 ml Acetonitril gibt man bei 20°C auf einmal 136,8 g (0,56 Mol) 4-(2-Chlorphenoxy)-5,6-difluorpyrimidin, wobei die Temperatur auf 31°C steigt. Es wird noch 6 Stunden bei 50°C und dann ohne weitere Wärmezufuhr über Nacht gerührt, wobei sich die Mischung abkühlt. Das Reaktionsgemisch wird zu 2,3 l Eiswasser gegeben und 5 Stunden gerührt, wobei das Produkt auskristallisiert. Dieses wird abgesaugt und mit 0,57 l Wasser portionsweise nachgewaschen. Man erhält 260 g (97,8% der Theorie) 3-{1-[2-(4-<2-Chlorphenoxy>-5-fluorpyrimid-6-yloxy)-phenyl]-1-(methoximino)-methyl}-5,6-dihydro-1,4,2-dioxazin vom Schmelzpunkt 75°C

### Beispiel 2

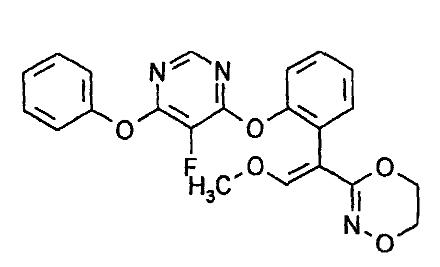

### Verfahren a)

Zu einer Lösung von 0,7 g (0,00298 Mol) 3-[1-(2-Hydroxyphenyl)-2-methoxyethen-1-yl]-5,6-dihydro-1,4,2-dioxazin und 0,6 g (0,00288 Mol) 4-Phenoxy-5,6-difluorpyrimidin in 10 ml Dimethylformamid gibt man bei 0°C 0,119 g Natriumhydrid (60%ige Suspension in Mineralöl) zu und rührt hierauf 12 Stunden bei 20°C. Man gießt das Reaktionsgemisch auf Wasser und extrahiert mit Essigsäureethylester. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (82 % der Theorie) 3-{1-[2-(4-Phenoxy-5-fluorpyrimid-6-yloxy)-phenyl]-2-methoxyethen-1-yl}-5,6-dihydro-1,4,2-dioxazin.

¹H-NMR-Spektrum (CDCl₃/TMS): δ = 3,678 (3H); 4,056/4,069/4,083 (2H); 4,300/4,314/4,328 (2H); 6,891 (1H); 7,199 - 7,475 (9H); 8,063 (1H) ppm.

### Beispiel 3

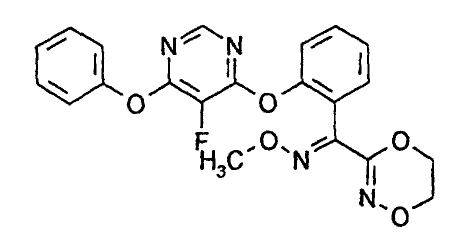

### Verfahren b)

Eine Mischung aus 124,1 g (0.333 Mol) 3-{1-[2-(4,5-Difluorpyrimid-6-yloxy)-phenyl]-1-(methoximino)-methyl}-5,6-dihydro-1,4,2-dioxazin, 31,3 g (0.333 Mol) Phenol, 46 g (0.333 Mol) Kaliumcarbonat, sowie 3,3 g Kupfer-(I)-chlorid in 1 l Dimethylformamid wird über Nacht bei 100°C gerührt. Nach Abkühlen auf 20°C wird das Lösungsmittel bei vermindertem Druck abdestilliert. Der Rückstand wird mit Essigsäureethylester aufgenommen und mehrmals mit Wasser gewaschen. Die organische Phase wird über Natriumsulfat getrocknet und wiederum bei vermindertem Druck eingeengt. Der Rückstand wird mit Hexan/Aceton (7:3) an Kieselgel chromatografiert. Man erhält 112,4 g (97% der Theorie) 3-{1-[2-(4-Phenoxy-5-fluorpyrimid-6-yloxy)-phenyl]-1-(methoximino)-methyl}-5,6-dihydro-1,4,2-dioxazin vom Schmelzpunkt 110°C.

Analog den Beispielen 1 bis 3, sowie entsprechend den Angaben in der allgemeinen Verfahrensbeschreibung, werden die in der nachstehenden Tabelle 1 genannten Verbindungen der Formel (I) erhalten.

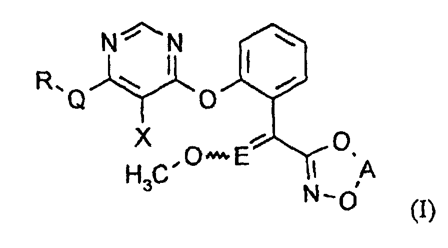

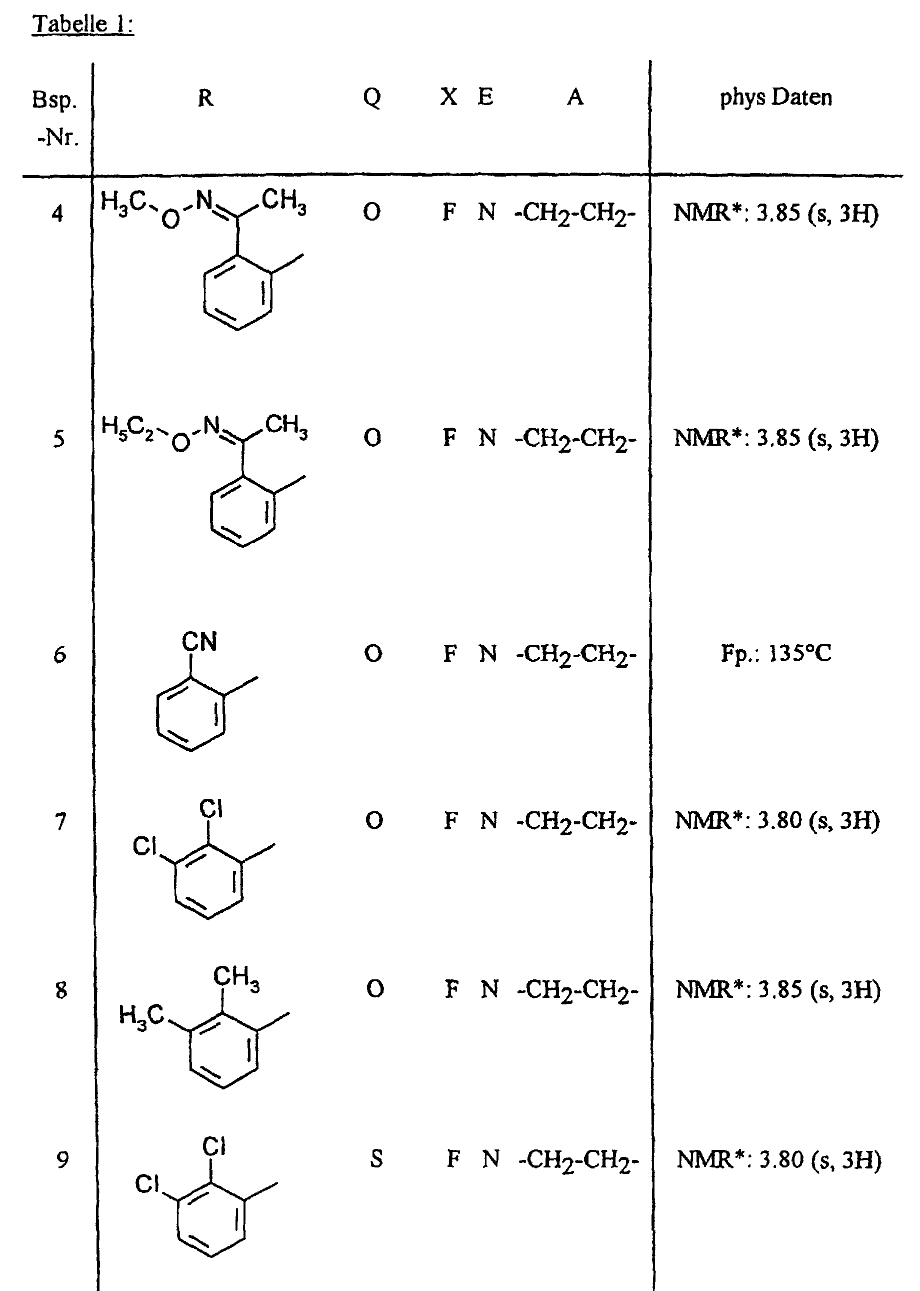

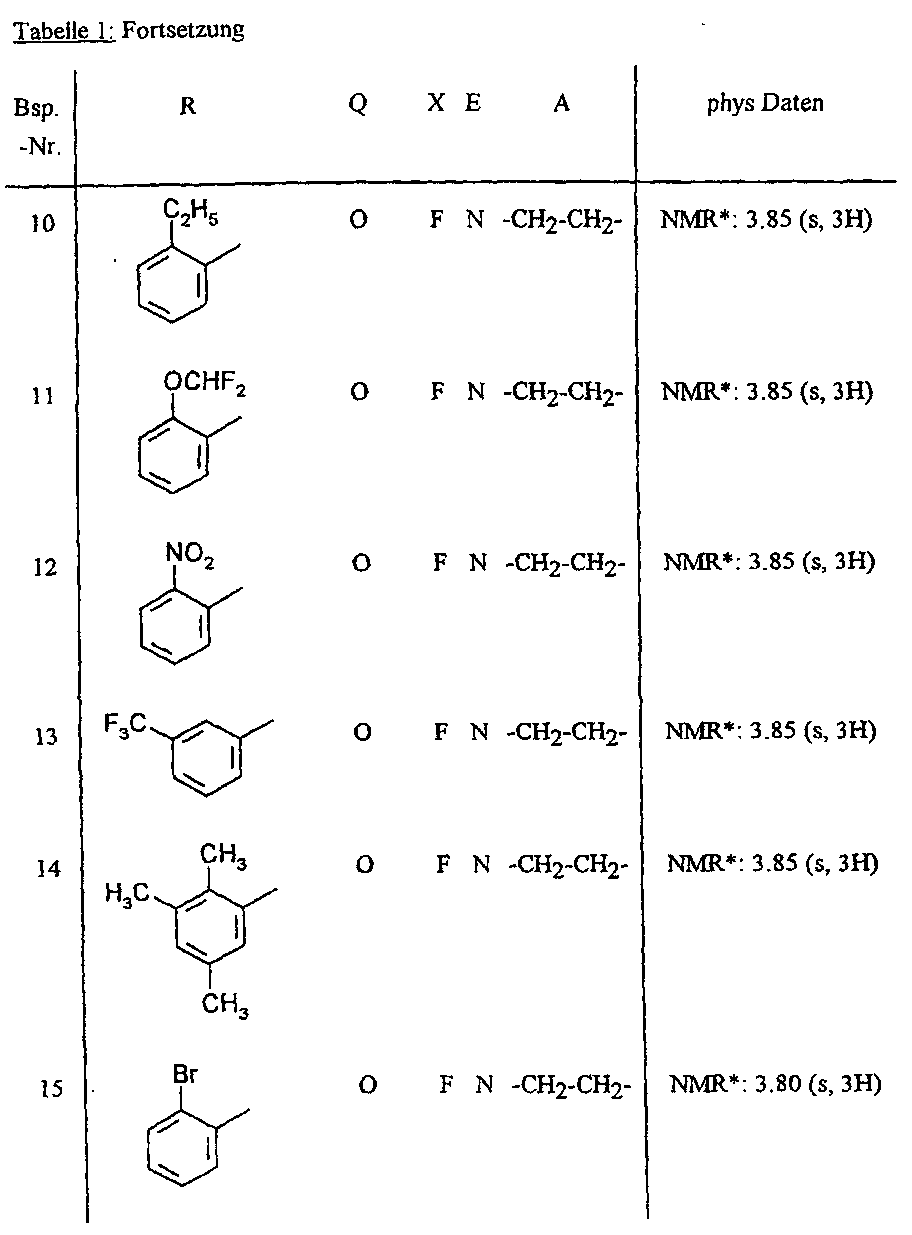

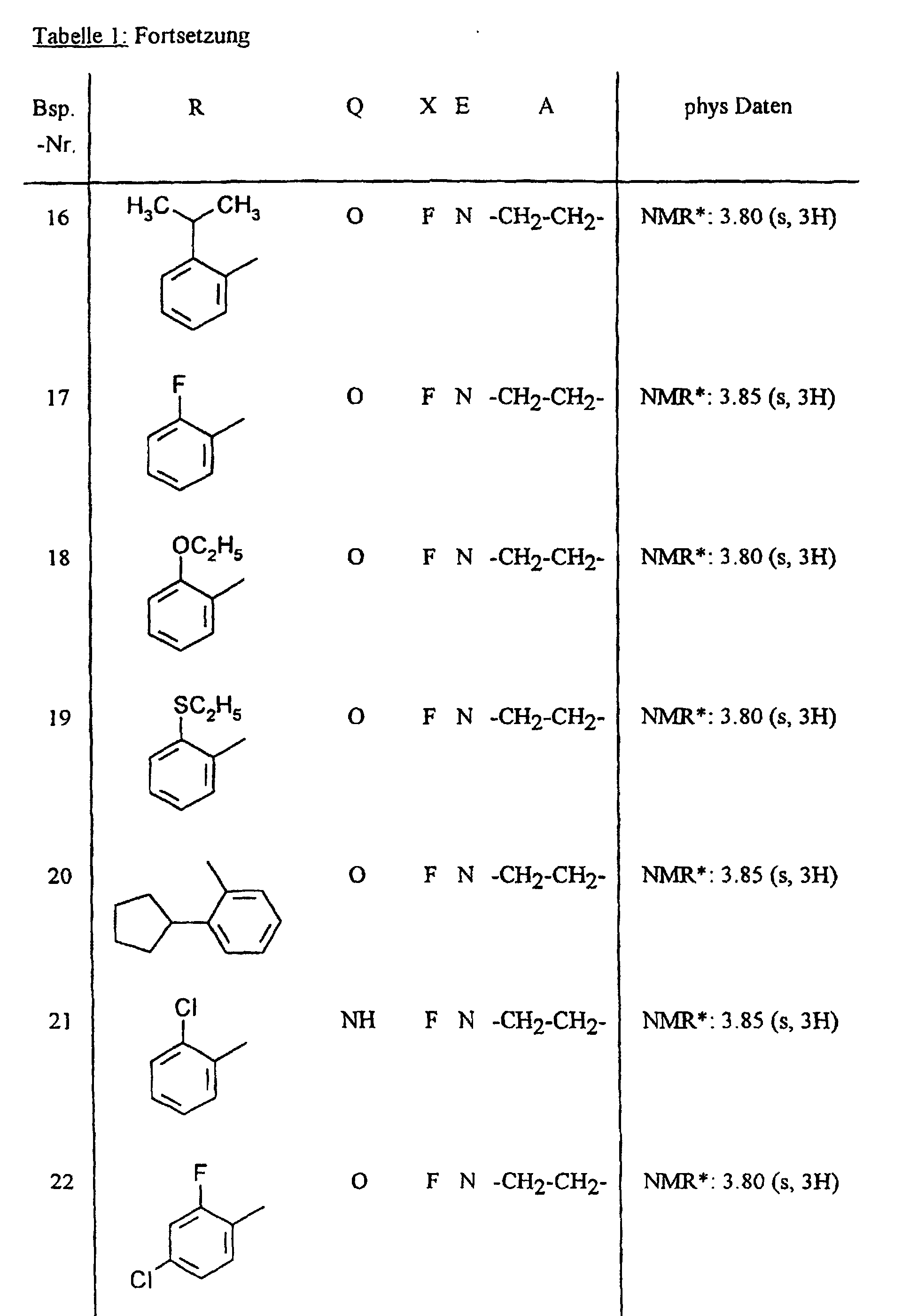

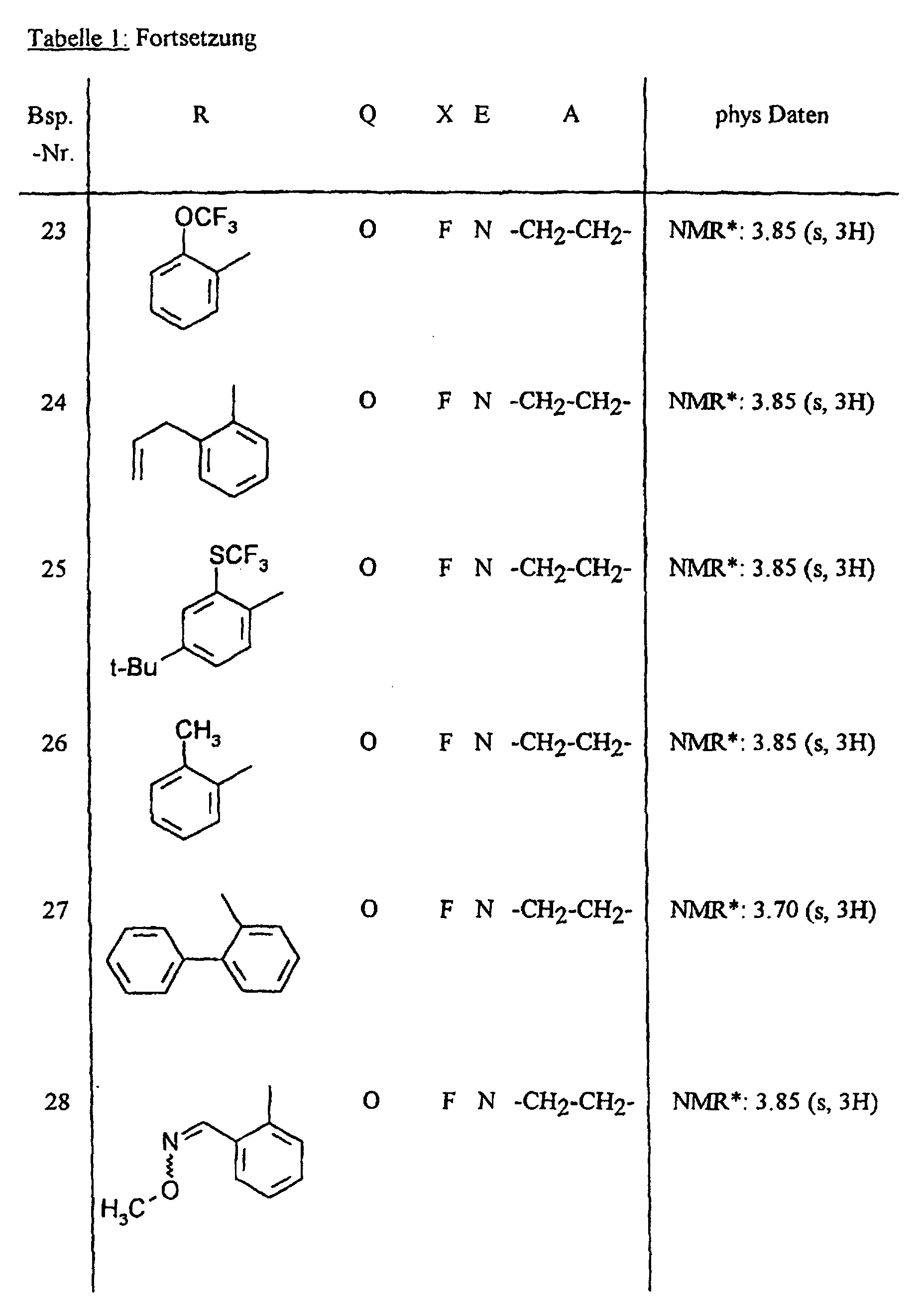

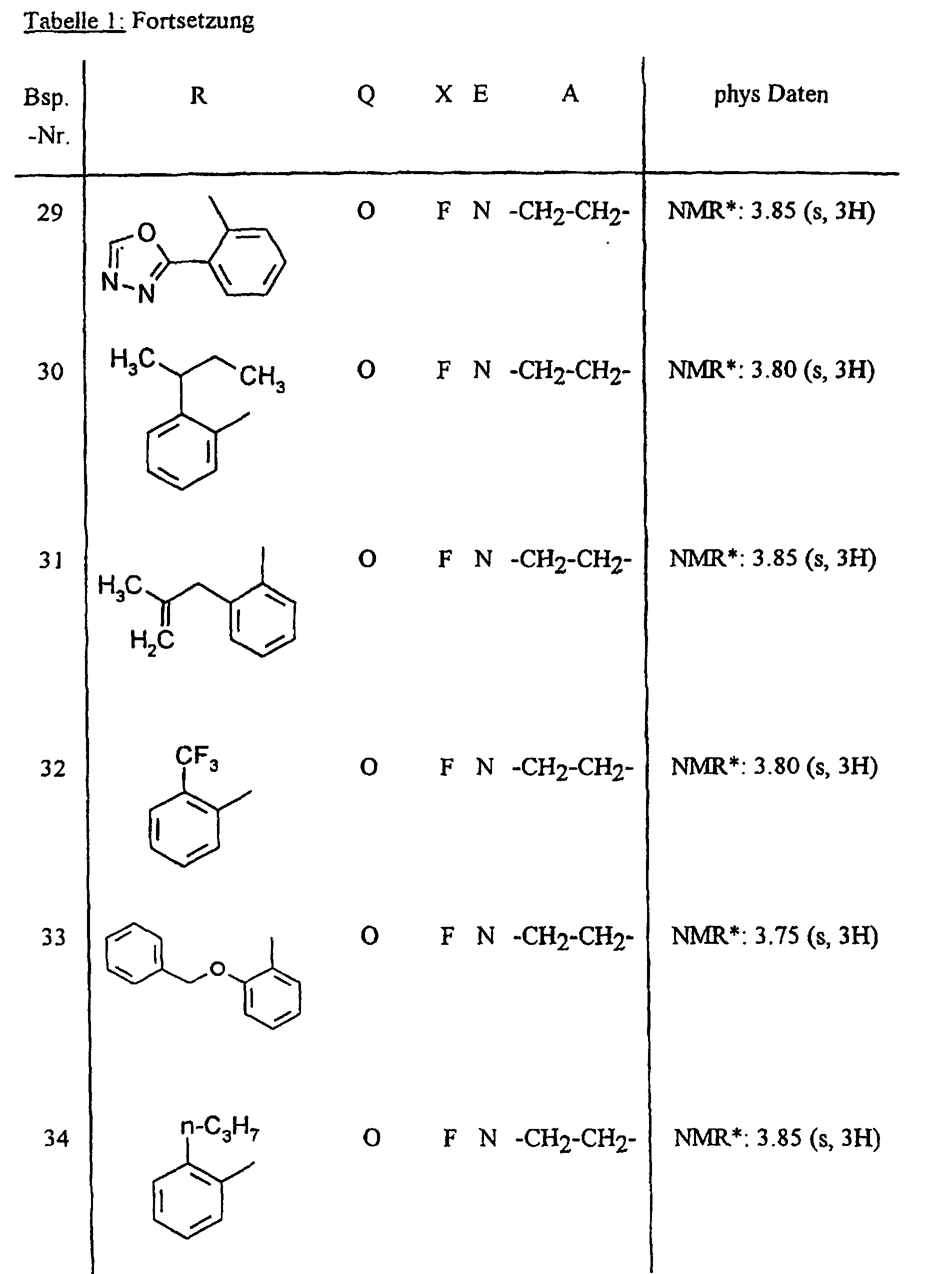

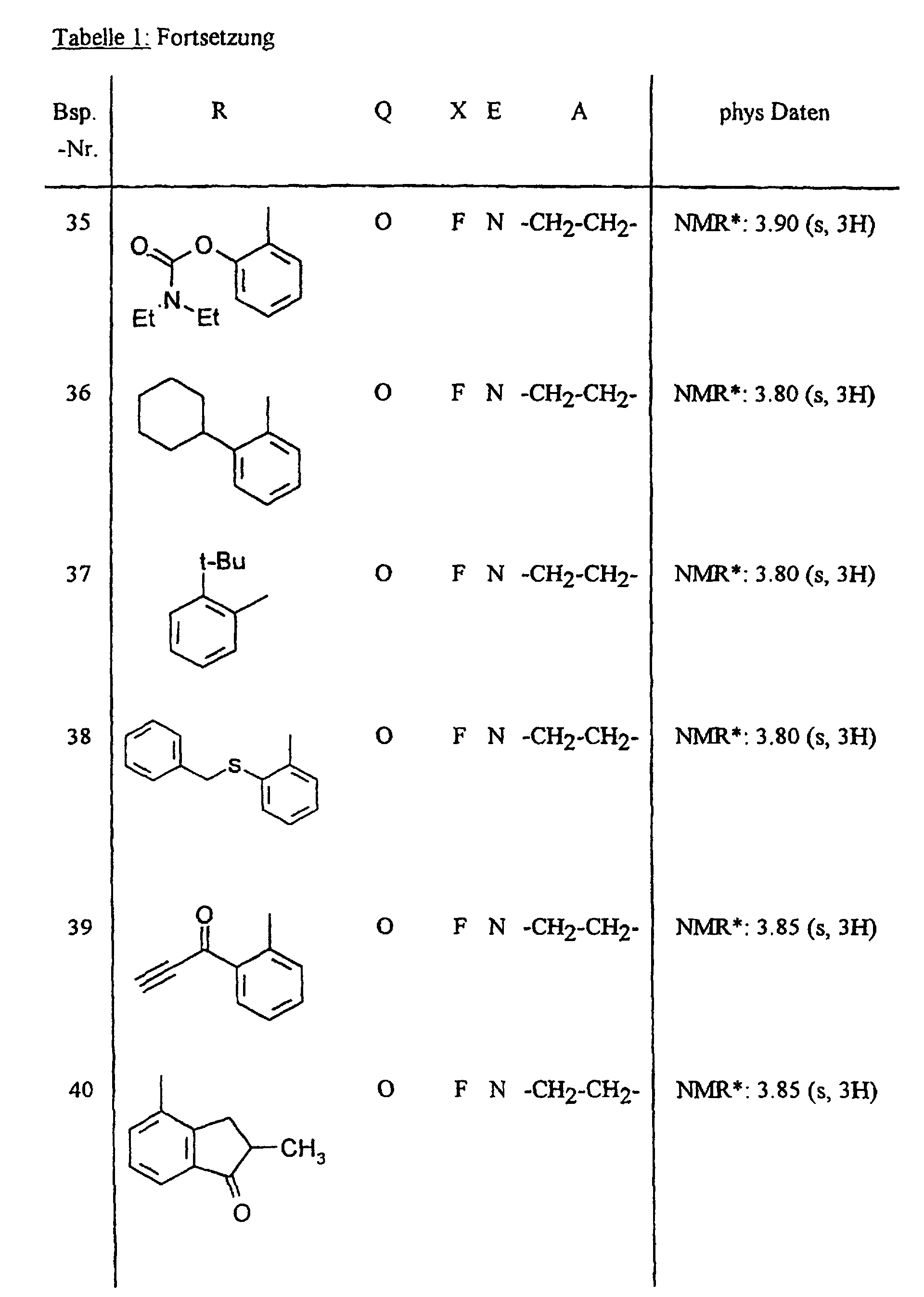

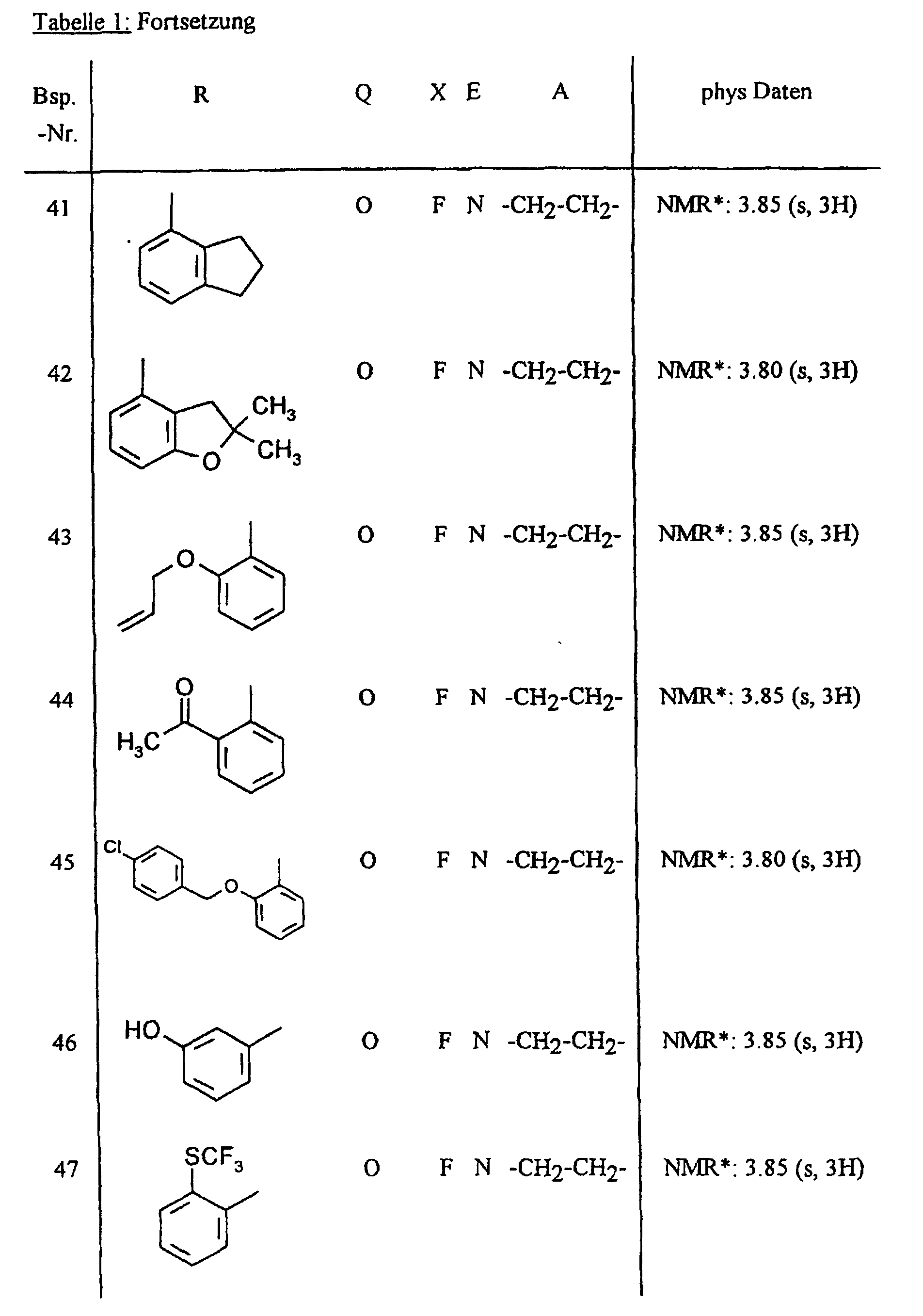

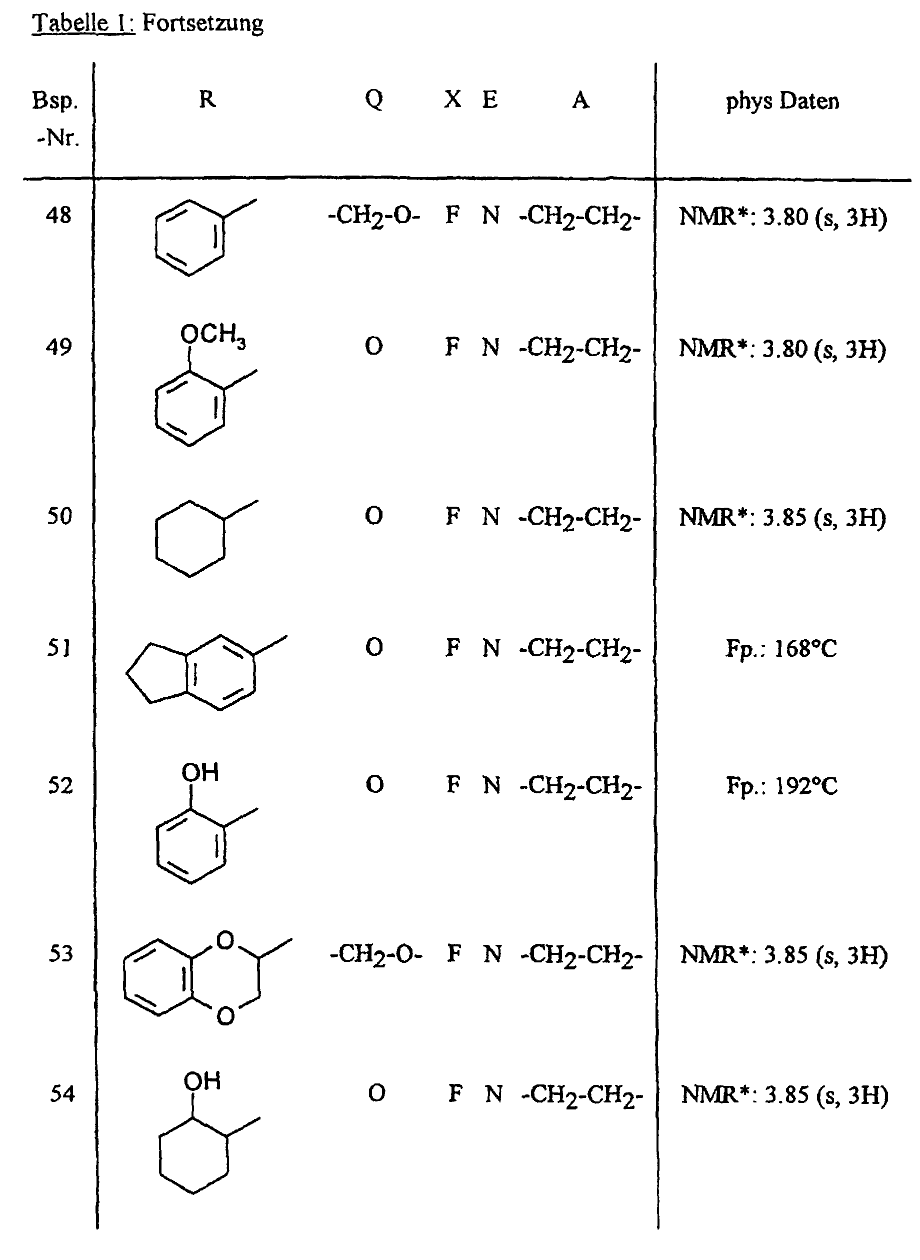

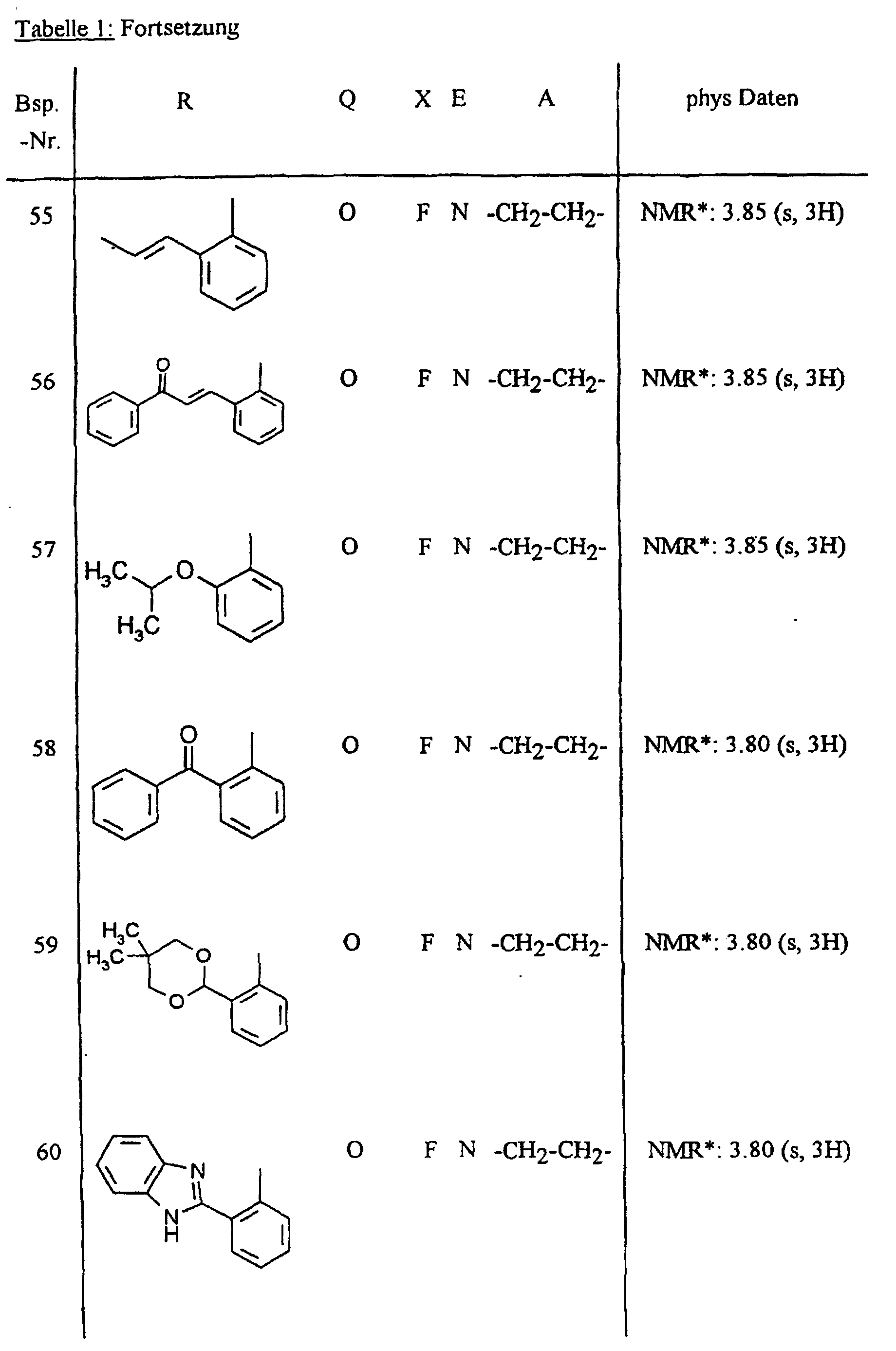

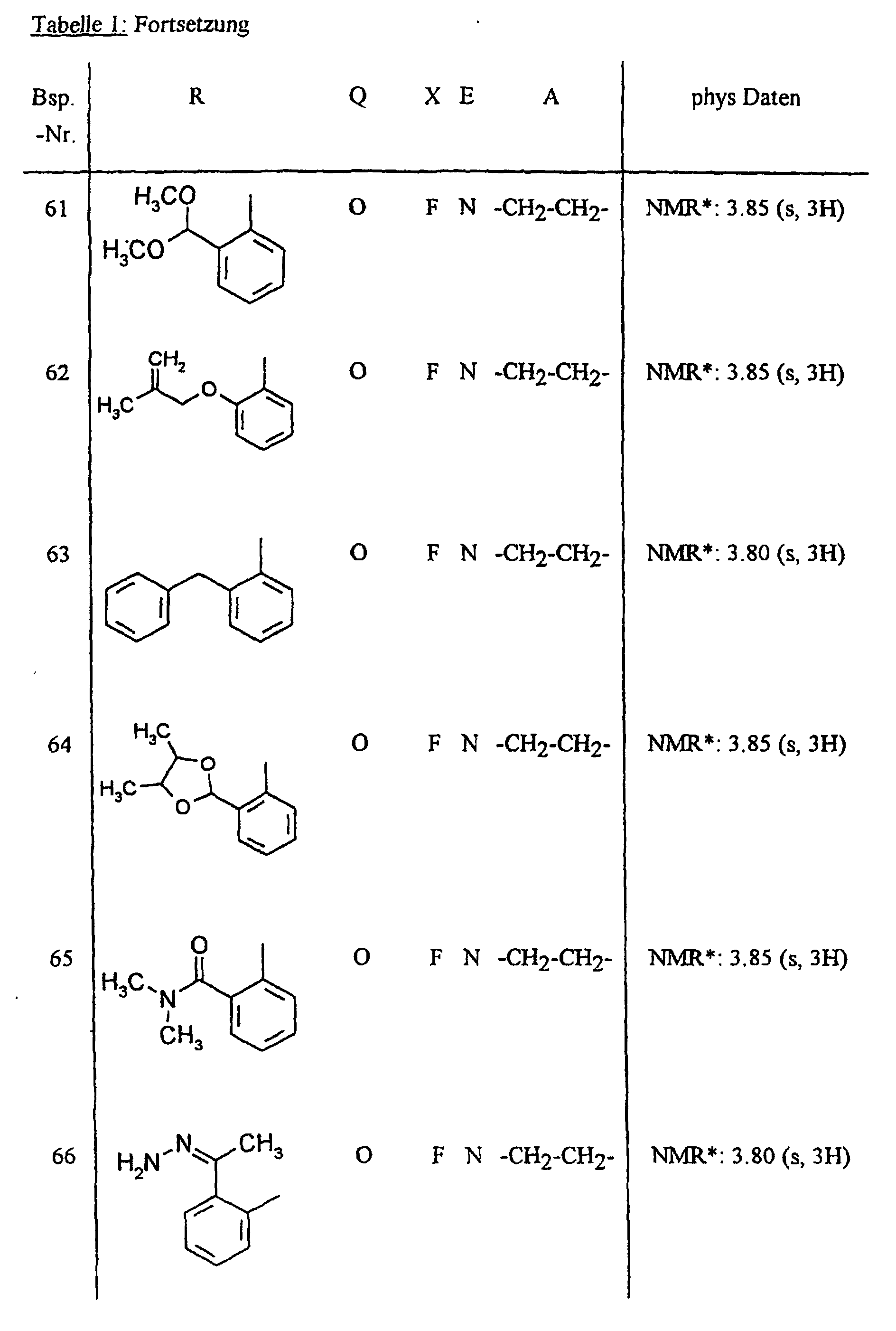

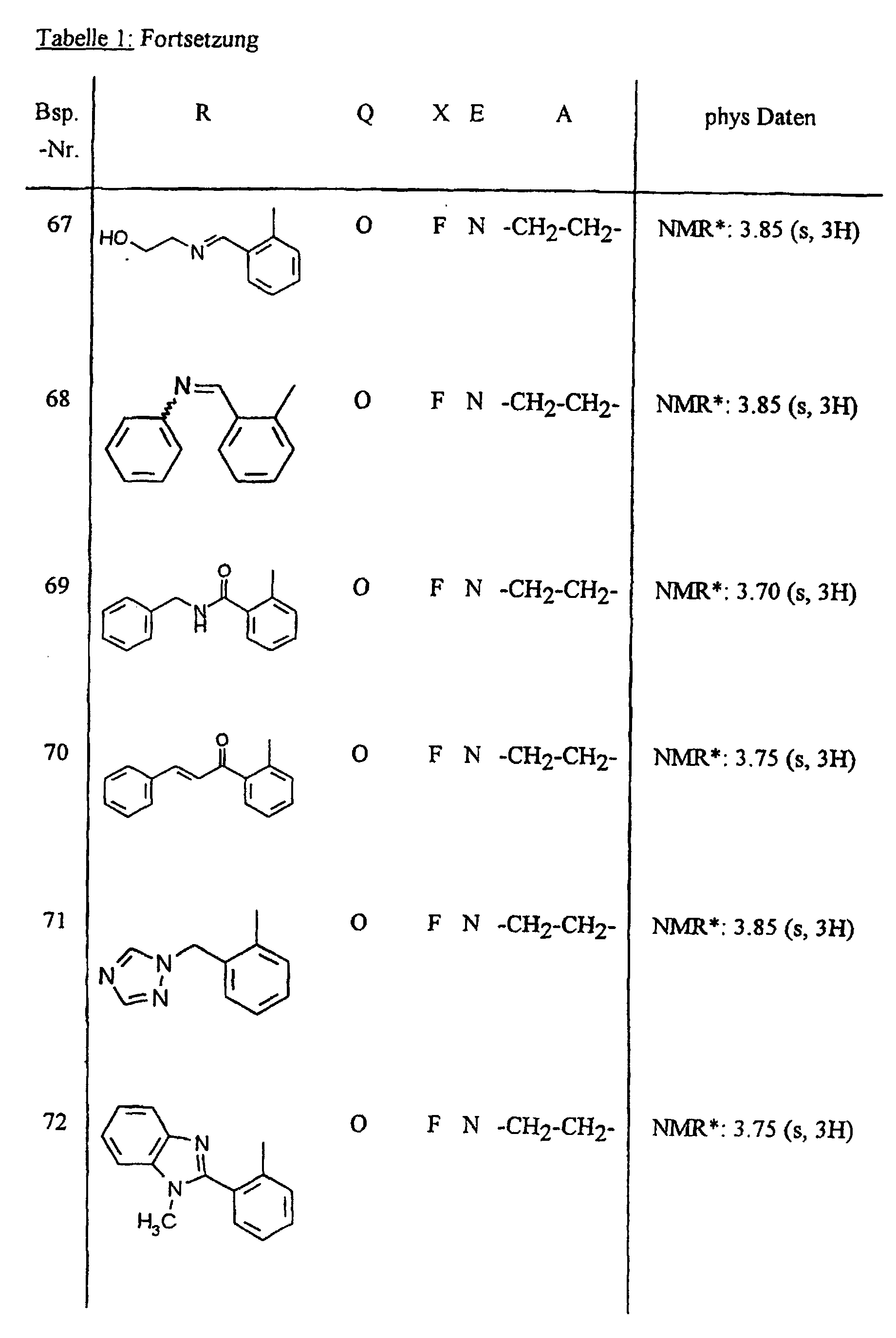

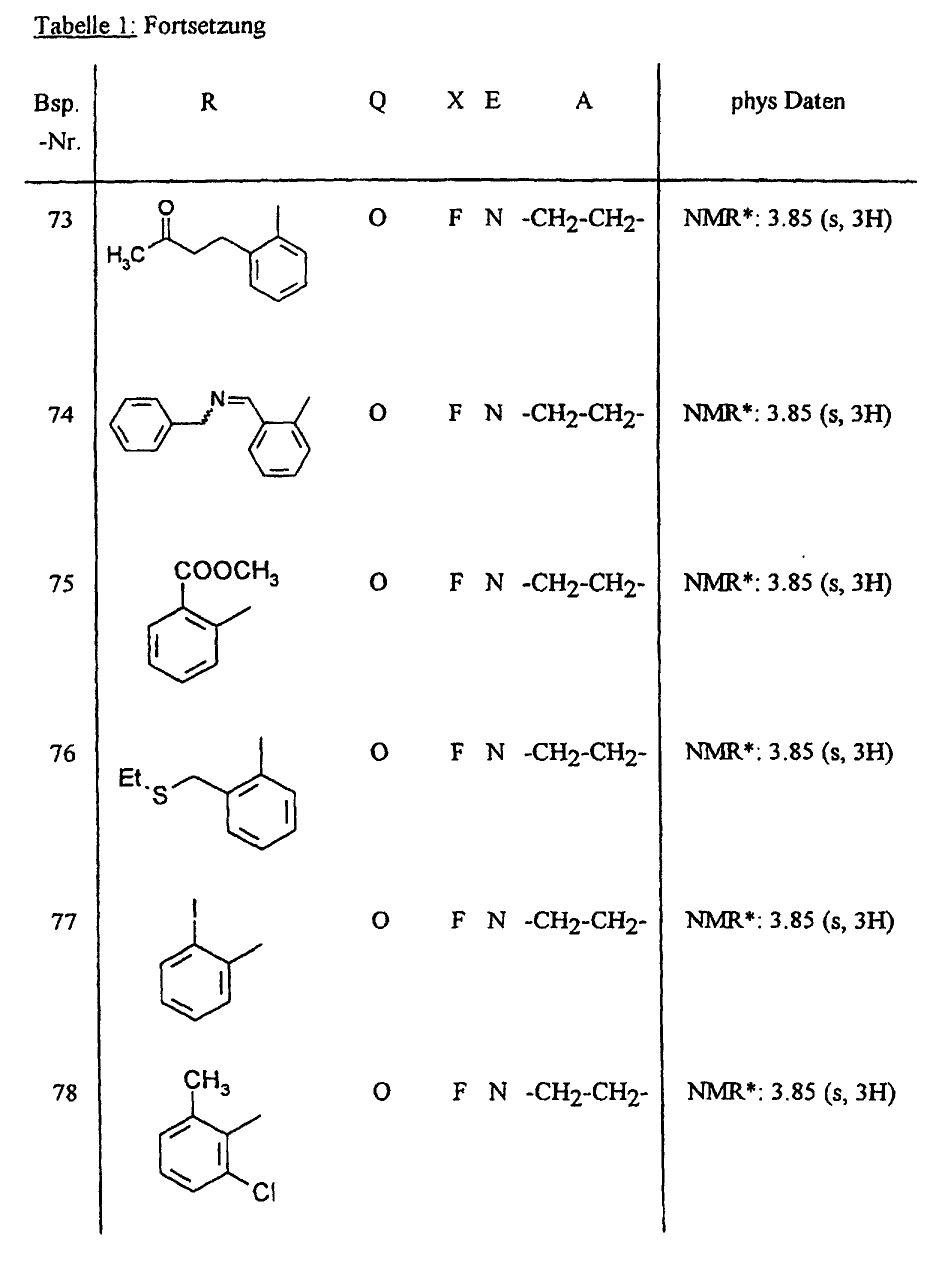

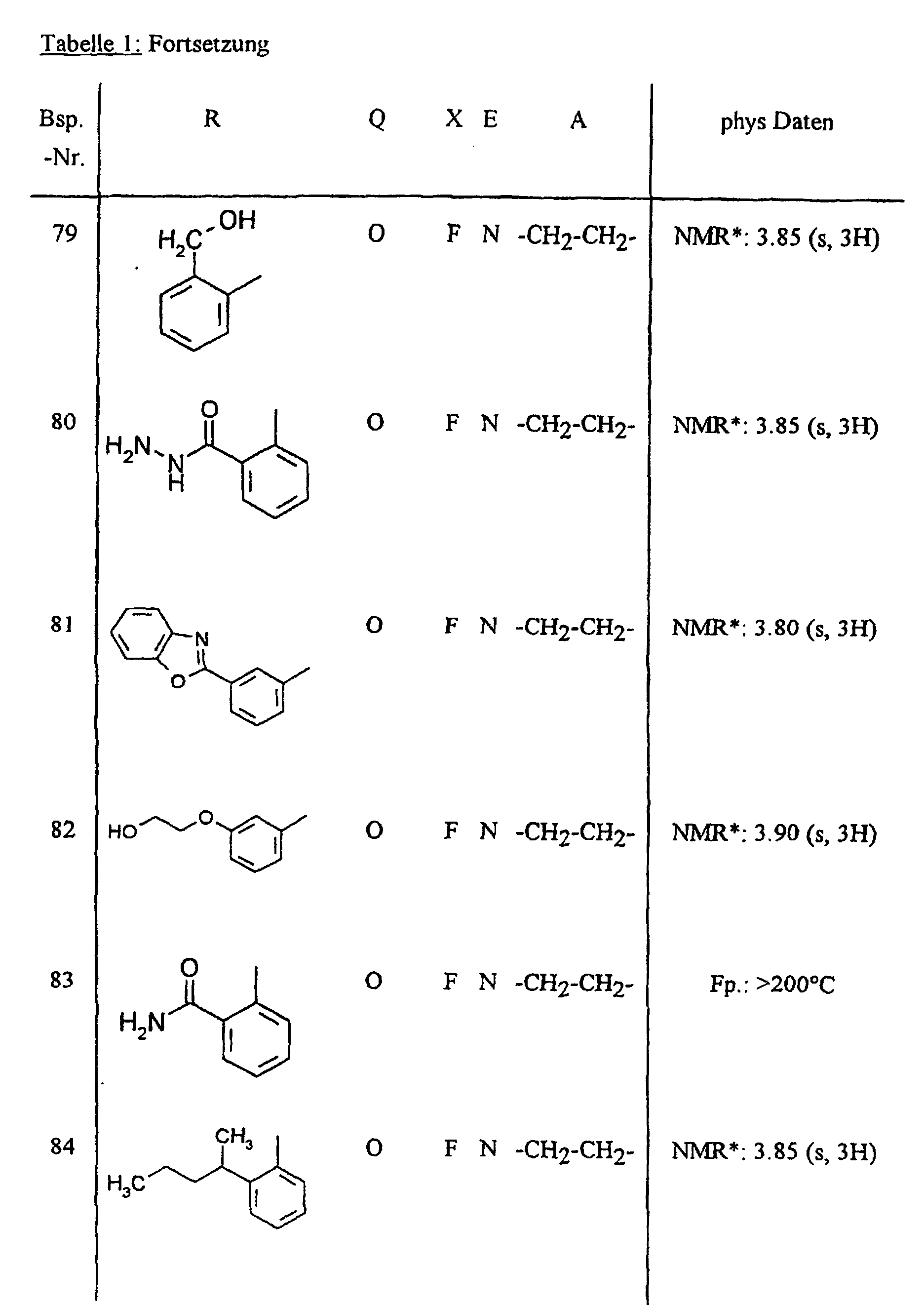

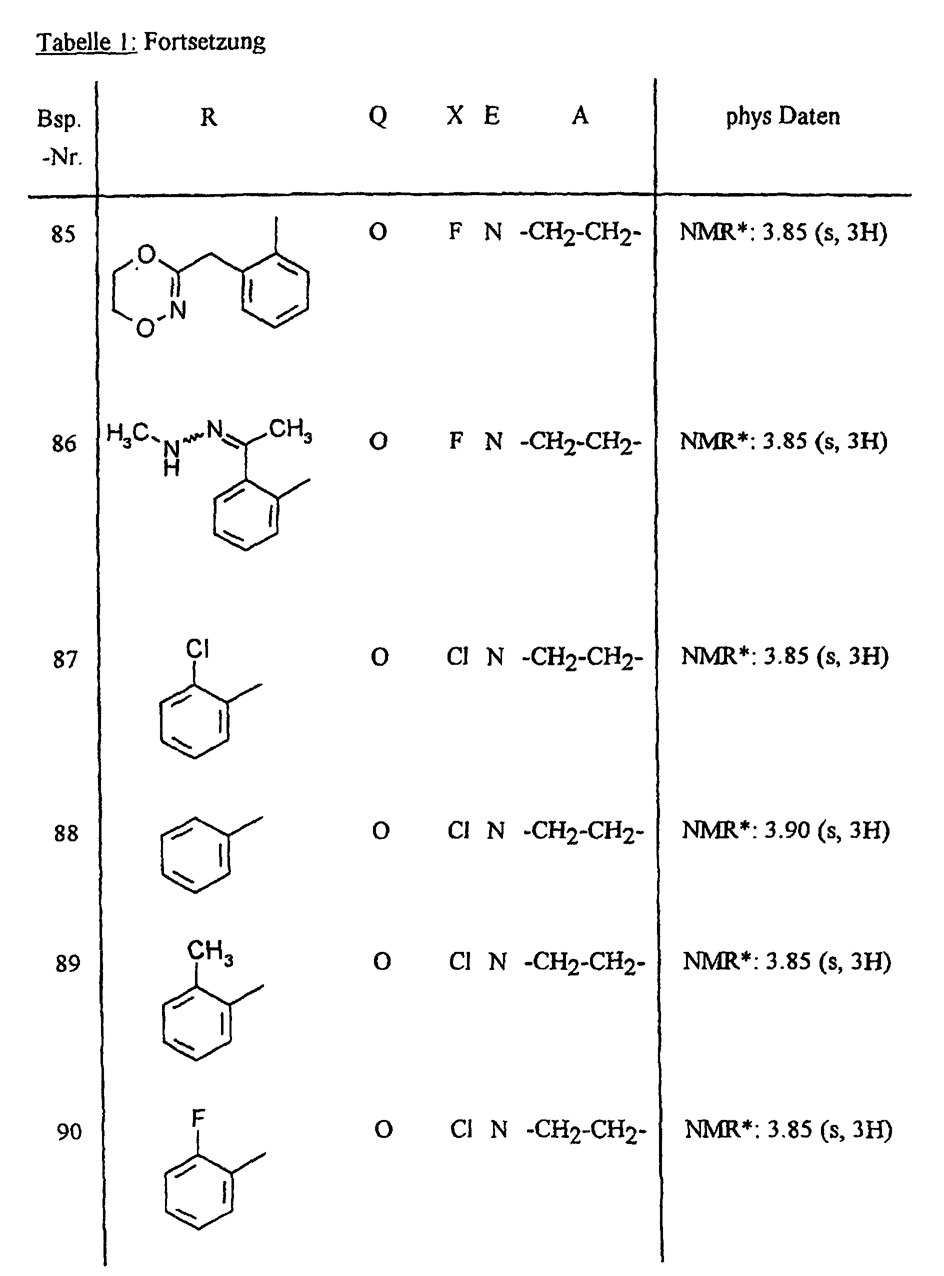

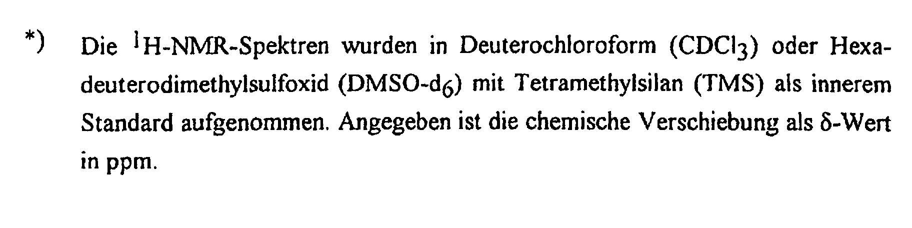

### Herstellung der Ausgangsverbindungen nach Formel (IIa):

### Beispiel (IIa-1):

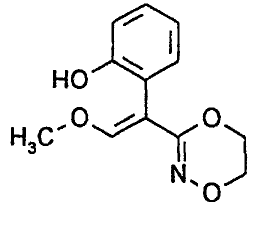

### Verfahren a-1)

7,5 g (0,0235 Mol) 3-{1-[2-(Tetrahydropyran-2-yloxy)-phenyl]-2-methoxyethen-1-yl}-5,6-dihydro-1,4,2-dioxazin werden in 20 ml Methanol mit 0,15 g saurem Ionenaustauscherharz 16 Stunden bei 20°C gerührt. Das Ionenaustauscherharz wird abfiltriert und das Filtrat bei vermindertem Druck eingeengt. Der Rückstand wird mit Cyclohexan/Essigester (1:1) an Kieselgel chromatografiert. Man erhält 1 g (18 % der Theorie) 3-[1-(2-Hydroxyphenyl)-2-methoxyethen-1-yl]-5,6-dihydro-1,4,2-dioxazin.

¹H-NMR-Spektrum (CDCl₃/TMS): δ = 3,794 (3H); 4,102-4,130 (2H); 4,383-4,411 (1H); 6,846 1H); 6,885-6,994 (2H); 7,157-7,260 (2H) ppm.

### Herstellung von Ausgangsstoffen nach Formel (III)

### Beispiel (III-1)

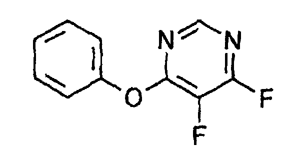

Eine Lösung von 42,4 g (0,45 Mol) Phenol und 50,4 g (0,45 Mol) Kalium-tert.butylat in 400 ml Tetrahydrofuran tropft man bei 0°C zu einer Lösung von 80 g (0,6 Mol) 4,5,6-Trifluorpyrimidin in 1l Tetrahydrofuran. Nach vollendeter Zugabe rührt man 30 Minuten bei 0°C, gießt dann das Reaktionsgemisch auf Wasser und extrahiert mit Essigsäureethylester. Man trocknet die organische Phase über Natriumsulfat, engt im Vakuum ein und verrührt den Rückstand mit tiefsiedendern Petrolether. Man erhält 63,8 g (68,1 % der Theorie) 4-Phenoxy-5,6-difluorpyrimidin vom Schmelzpunkt 65 - 66°C.

Analog Beispiel (III-1) werden die Verbindungen der Formel 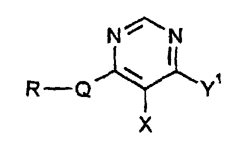 erhalten.

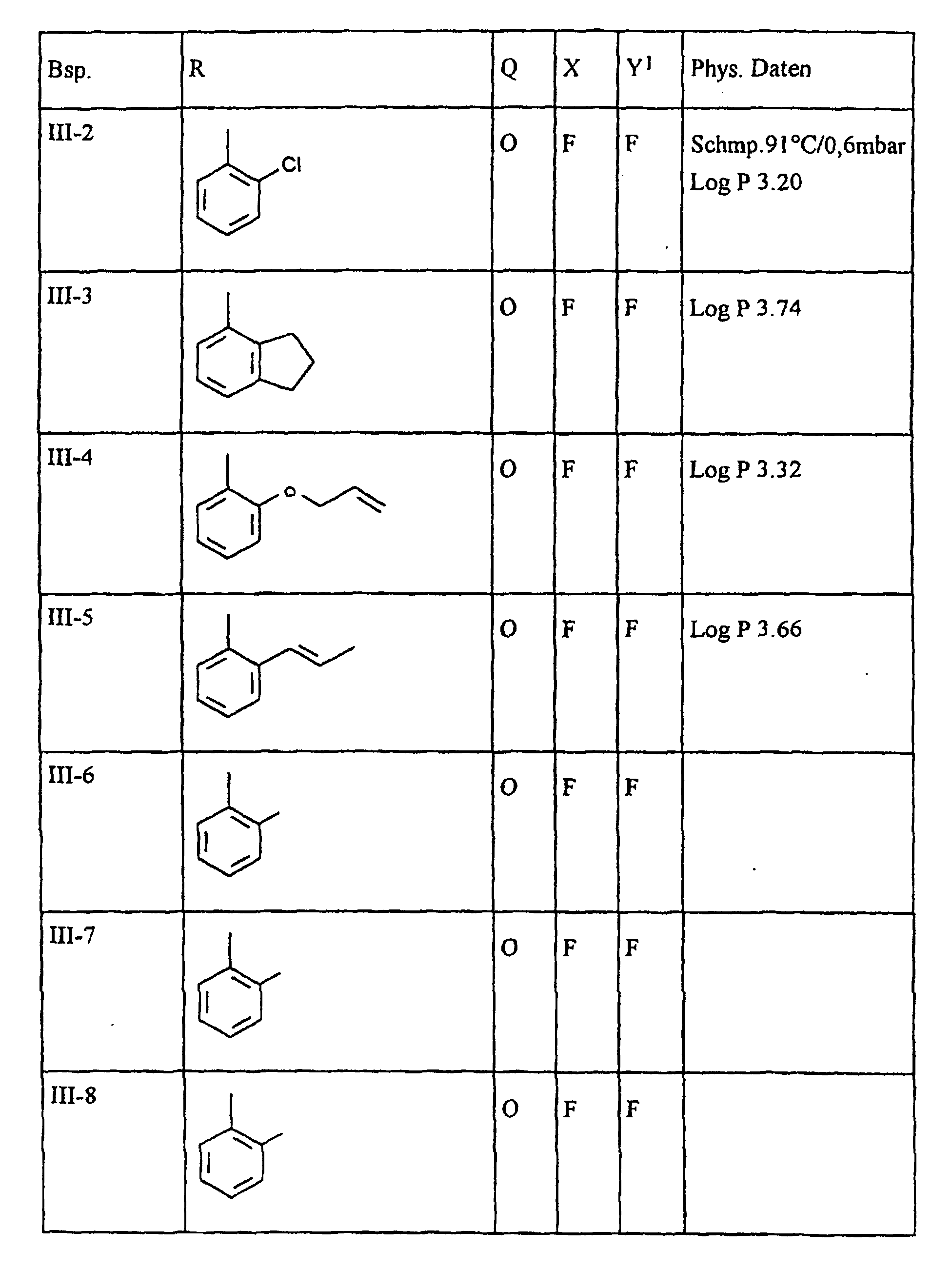

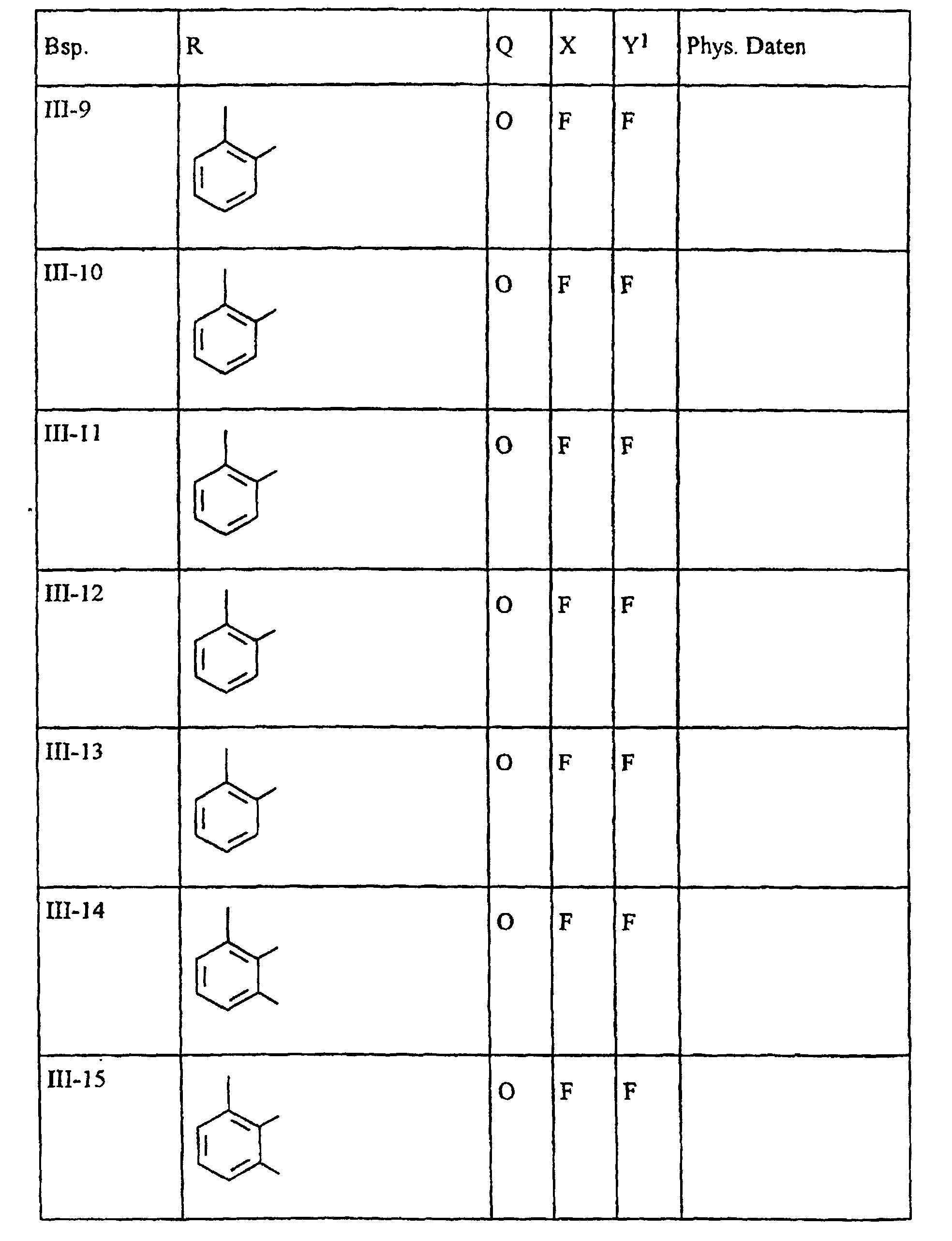

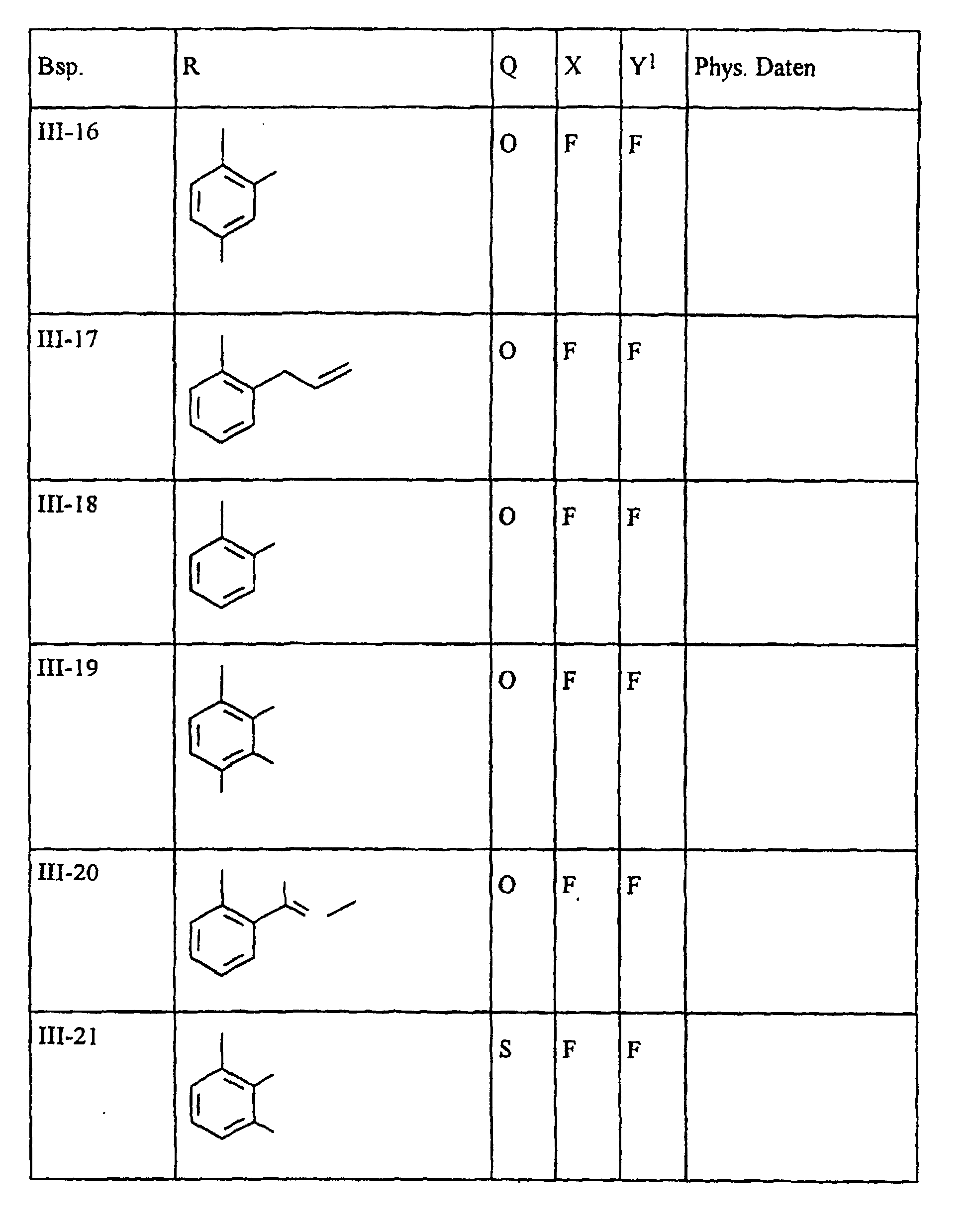

### Herstellung der Ausgangsstoffe nach Formel (IV):

### Beispiel (IV-1):

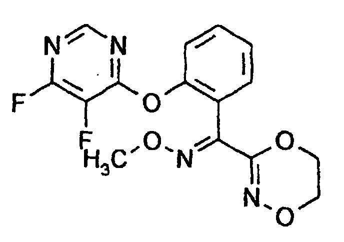

### Verfahren b-1)

Zu einer Lösung von 47,2 g (0,2 Mol) 3-[1-(2-Hydroxyphenyl)-1-(methoximino)-methyl]-5,6-dihydro-1,4,2-dioxazin (WO-A 9504728) in 1 l Tetrahydrofuran werden bei 0°C zunächst 29,3 g (0,22 Mol) 4,5,6-Trifluorpyrimidin und anschließend 6,0 g (0,2 Mol) Natriumhydrid (80%ige Suspension in Mineralöl) in kleinen Portionen gegeben. Die Mischung wird 3 Stunden bei 0°C und anschließend über Nacht ohne weitere Kühlung gerührt. Der Rückstand wird mit Essigsäureethylester aufgenommen und mehrmals mit Wasser gewaschen. Die organische Phase wird über Natriumsulfat getrocknet und wiederum bei vermindertem Druck eingeengt, wobei ein zähes Öl zurückbleibt, das langsam kristallisiert. Man erhält 68,7 g (98% der Theorie) 3-{1-[2-(4,5-Difluorpyrimid-6-yloxy)-phenyl]-1-(methoximino)-methyl}-5,6-dihydro-1,4,2-dioxazin vom Schmelzpunkt 98°C.

Analog Beispiel (IV-1) wurde 3-{1-[2-(5-Chlor-4-fluorpyrimid-6-yloxy)-phenyl]-1-(methoximino)-methyl}-5,6-dihydro-1,4,2-dioxazin, Beispiel (IV-2), als hockviskoses Öl erhalten.

¹H-NMR-Spektrum (CDCl₃/TMS): δ = 3,80 (s, 3H) ppm.

### Herstellung der Vorprodukte nach Formel (VI):

### Beispiel (VI-1):

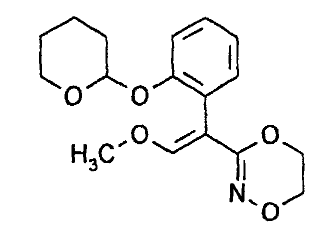

### Verfahren a-2)

Eine Mischung aus 31,2 g (0,091 Mol) Methoxymethylen-triphenyl-phosphoniumchlorid und 10,2 g (0,091 Mol) Kalium-tert.-butylat in 100 ml Tetrahydrofuran wird 20 Minuten bei 20°C gerührt. Dann gibt man eine Lösung von 13,3 g (0,0457 Mol) 3-[2-(Tetrahydropyran-2-yloxy)-benzoyl]-5,6-dihydro-1,4,2-dioxazin in 100 ml Tetrahydrofuran zu und erhitzt 12 Stunden unter Rückfluß zum Sieden. Man engt die Mischung bei vermindertem Druck ein und verteilt den Rückstand zwischen Wasser und Essigsäureethylester. Die organische Phase wird abgetrennt und über Natriumsulfat getrocknet. Der Rückstand wird mit Cyclohexan/Essigester (1:1) an Kieselgel chromatografiert. Man erhält 9,2 g (63 % der Theorie) 3-{1-[2-(Tetrahydropyran-2-yloxy)-phenyl]-2-methoxyethen-1-yl}-5,6-dihydro-1,4,2-dioxazin.

¹H-NMR-Spektrum (CDCl₃/TMS): δ = 1,5 - 1,92 (6 H); 3,5 - 4,0 (2H); 3,730 (3H); 4,056 - 4,111 (2H); 4,295 - 4,325 (2H); 5,410/5,420 (1H); 6,963 (1H); 6,950 - 7,461 (4H) ppm.

### Herstellung der Vorprodukte nach Formel (VII)

### Beispiel (VII-1):

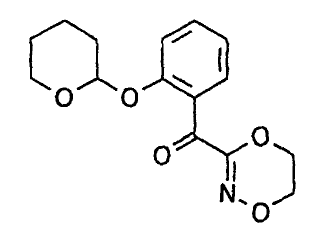

### Verfahren a-3)

5 g (0,0193 Mol) 1-(Tetrahydropyran-2-yloxy)-2-brombenzol (Synthesis 1987, Seite 951) werden in 20 ml Tetrahydrofuran gelöst und auf - 40 °C gekühlt. Bei dieser Temperatur tropft man erst 10,8 g (0,0388 Mol) n-Butyllithium (23%ige Lösung in Hexan), dann eine 50 %igen Lösung von 7,2 g (0,0195 Mol) 1-(5,6-Dihydro-1,4,2-dioxazin-3-yl)-1-(pyrrolidin-1-yl)-methanon in Tetrahydrofuran zu und rührt 10 Minuten bei -40 °C. Nun tropft man eine Lösung von 4,2 g (0,0785 Mol) Ammoniumchlorid in 25 ml Wasser zu, versetzt mit Diethylether, trennt die organische Phase ab und extrahiert die wäßrige Phase mehrmals mit Diethylether. Die vereinigten organischen Phasen werden über Natriumsulfat getrocknet und bei vermindertem Druck eingeengt. Der Rückstand kristallisiert beim Verreiben mit Pentan. Man filtriert ab und wäscht die Kristalle zweimal mit 5 ml Diisopropylether. Man erhält 2,4 g (35,8 % der Theorie) 3-[2-(Tetrahydropyran-2-yloxy)-benzoyl]-5,6-dihydro-1,4,2-dioxazin (84% Gehalt nach HPLC-Analyse).

¹H-NMR-Spektrum (CDCl₃/TMS): δ = 1,565-1,954 (6H); 3,54-3,68 (1H); 3,78-4,0 (1H); 4,154-4,354 (2H); 4,448-4,510 (2H); 5,512 (1H); 7,004-7,056 (1H); 7,199/7,227 (1H); 7,408-7,463 (2H) ppm.

### Herstellung der Vorprodukte nach Formel (IX)

### Beispiel (IX-1):

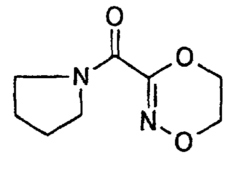

### Verfahren a-4)

Man löst 44,9 g (0,8 Mol) Kaliumhydroxid in 107 ml Methanol und 27,8 g (0,4 Mol) Hydroxylammoniumchlorid in 180 ml Methanol, vereint beide Lösungen bei 35°C bis 40°C. Dann gibt man bei 10 bis 20°C 34,2 g (0,2 Mol) Oxo-pyrrolidin-1-ylessigsäure-ethylester (EP-A 469889) zu und rührt 30 Minuten bei 20°C. Sodann gibt man 27,6 g (0,2 Mol) Kaliumcarbonat und 169,1 g (0,9 Mol) Dibromethan zu und kocht 4 Stunden unter Rückfluß. Man filtriert von den Salzen ab, engt das Filtrat bei vermindertem Druck ein, nimmt den Rückstand in 600 ml Essigsäureethylester auf und wäscht die organische Phase sukzessive mit 50 ml gesättigter, wäßriger Natriumchlorid-Lösung und mit 50 ml halbgesättigter, wäßriger Natriumchlorid-Lösung. Man trocknet die organische Phase über Natriumsulfat, destilliert das Lösungsmittel im Vakuum ab, zuletzt im Hochvakuum bei 2 Torr und 60°C. Man erhält 20,9 g (52,2 % der Theorie) 1-(5,6-Dihydro-1,4,2-dioxazin-3-yl)-1-(pyrrolidin-1-yl)-methanon (92% Gehalt nach HPLC-Analyse).

¹H-NMR-Spektrum (CDCl₃/TMS): δ = 1,841-1,978 (4H); 3,491-3,547 (2H); 3,709-3,793 (2H); 4,166-4,194 (2H); 4,460-4,487 (2H) ppm.

### Herstellung eines Vorproduktes nach Formel (XIII)

### Beispiel (XIII-1)

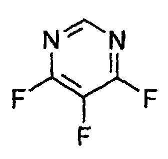

Aus einer Mischung von 609 g Kaliumfluorid in 2,3 l Sulfolan werden zur Trocknung 500 ml Flüssigkeit bei 145°C und 20 mbar abdestilliert. Anschließend werden 1054 g 5-Chlor-4,6-difluorpyrimidin (DE-A 3843558) und 25 g Tetraphenylphosphoniumbromid zugegeben, 5 bar Stickstoff aufgedrückt und 24 Stunden bei 240°C gerührt, wobei der Druck bis 11 bar steigt. Die Reaktionsmischung wird auf 80°C gekühlt und entspannt. Nun wird die Mischung bei Normaldruck wieder langsam erhitzt, wobei das Produkt abdestilliert. Hat die Sumpftemperatur 200°C erreicht, wird der Druck auf 150 mbar vermindert, um die Destillation zu beschleunigen und um weiteres Produkt zu erhalten. Insgesamt erhält man 664 g (70,7 % der Theorie) 4,5,6-Trifluorpyrimidin vom Siedepunkt 86 bis 87°C.

### Anwendungsbeispiele

### Beispiel A

### Phytophthora-Test (Tomate) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Bei diesem Test zeigen z.B. die Verbindungen (1), (2), (3), (6), (8), (10), (17), (40), (41), (43), (47), (49), (55), (63), (76), (77) und (78) der Herstellungsbeispiele bei einer Wirkstoffkonzentration von 100 ppm einen Wirkungsgrad von mehr als 90 %.

### Beispiel B

### Plasmopara-Test (Reben) / protektiv

| | |
|---|---|
| Lösungsmittel | 4,7 GewichtsteilAceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

Bei diesem Test zeigen z.B. die Verbindungen der Herstellungsbeispiele (1), (2), (3), (4), (5), (6), (8), (10), (11), (14), (15), (16), (17), (18), (22), (23), (26), (28), (32), (33), (38), (39), (40), (41), (43), (45), (47), (48), (49), (53), (55), (63), (76), (77) und (78) bei einer beispielhaften Wirkstoffkonzentration von 100ppm einen Wirkungsgrad bis zu 100%.

### Beispiel: C

### Sphaerotheca-Test (Gurke) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Bei diesem Test zeigen z.B. die Verbindungen (1), (2), (3), (8), (10), (13), (15), (17), (22), (23), (26), (28), (32), (37), (41), (47), (48) und (49) der Herstellungsbeispiele bei einer Wirkstoffkonzentration von 100 ppm einen Wirkungsgrad bis zu 100 %.

### Beispiel D

### Venturia-Test (Apfel) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Bei diesem Test zeigen z.B. die Verbindungen der Herstellungsbeispiele (1), (2), (3), (4), (5), (6), (8), (10), (11), (13), (14), (15), (16), (17), (18), (22), (26), (28), (32), (33), (37), (38), (39), (41), (43), (45), (47), (48), (49), (53), (55), (63), (77) und (78) bei einer Wirkstoffkonzentration von 10 ppm einen Wirkungsgrad bis zu 100 %.

### Beispiel E

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei diesem Test zeigt z.B. die Verbindung (59) bei einer Wirkstoffaufwandmenge von 250 g/ha einen Wirkungsgrad von 100 % im Vergleich zur unbehandelten Kontrolle.

### Beispiel F

### Erysiphe-Test (Gerste) / kurativ

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht. Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei diesem Test zeigen z.B. die Verbindungen der Herstellungsbeispiele (1) und (6) bei einer Wirkstoffaufwandmenge von 250 g/ha einen Wirkungsgrad von 100 % im Vergleich zur unbehandelten Kontrolle.

### Beispiel: G

### Fusarium nivale (var. majus) - Test (Weizen)/protektiv

| | |
|---|---|
| Lösungsmittel | 10 Gewichtsteile N-Methyl-pyrrolidon |
| Emulator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge behandelt.

Nach Antrocknen der Spritzbrühe werden die Pflanzen mit einer Konidiensuspension von Fusarium nivale var. majus besprüht.

Die Pflanzen werden in einem Gewächshaus unter lichtdurchiässigen Inkubationshauben bei einer Temperatur von ca. 15°C und einer relativen Luftfeuchtigkeit von ca. 100% gehalten.

4 Tage nach der Inokulation erfolgt die Auswertung.

Bei diesem Test zeigen z.B. die Verbindungen der Herstellungsbeispiele (8), (11), (14), (15), (24), (33), (41), (42) und (55) bei einer beispielhaften Wirkstoffaufwandmenge von 250 g/ha einen Wirkungsgrad von 100 % im Vergleich zur unbehandelten Kontrolle.

### Beispiel: H

### Fusarium nivale (var. majus) - Test (Weizen)/kurativ

| | |
|---|---|
| Lösungsmittel | 10 Gewichtsteile N-Methyl-pyrrolidon |
| Emulator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidiensuspension von Fusarium nivale var. majus besprüht.

Die Pflanzen verbleiben 24 Stunden bei 15°C und 100 % rel. Luftfeuchtigkeit in einer Inkubationskabine. Anschließend besprüht man die Pflanzen mit der Wirkstoffzubereitung in der angegebenen Aufwandmenge.

Nach Antrocknen der Spritzbrühe verbleiben die Pflanzen in einem Gewächshaus unter lichtdurchlässigen Inkubationshauben bei einer Temperatur von ca. 15°C und einer relativen Luftfeuchtigkeit von ca. 100%.

4 Tage nach der Inokulation erfolgt die Auswertung.

Bei diesem Test zeigt z.B. die Verbindung des Herstellungsbeispiels (43) bei einer beispielhaften Wirkstoffaufwandmenge von 250 g/ha einen Wirkungsgrad von 100 % im Vergleich zur unbehandelten Kontrolle.

### Beispiel: I

### Fusarium nivale (var. nivale) - Test (Weizen)/protektiv

| | |
|---|---|
| Lösungsmittel | 10 Gewichtsteile N-Methyl-pyrrolidon |
| Emulator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge behandelt.

Nach Antrocknen der Spritzbrühe werden die Pflanzen mit einer Konidiensuspension von Fusarium nivale var. nivale besprüht.

Die Pflanzen werdenin einem Gewächshaus unter lichtdurchlässigen Inkubationshauben bei einer Temperatur von ca. 15°C und einer relativen Luftfeuchtigkeit von ca. 100 % gehalten.

4 Tage nach der Inokulation erfolgt die Auswertung.

Bei diesem Test zeigen z.B. die Verbindungen der Herstellungsbeispiel (10), (11), (15), (24), (32), (34), (43) und (55) bei einer beispielhaften Wirkstoffaufwandmenge von 250 g/ha einen Wirkungsgrad von 100 % im Vergleich zur unbehandelten Kontrolle.

### Beispiel: K

### Fusarium nivale (var. nivale) - Test (Weizen)/kurativ

| | |
|---|---|
| Lösungsmittel | 100 Gewichtsteile Dimethylformamid |
| Emulator | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidiensuspension von Fusarium nivale var. nivale besprüht.

Die Pflanzen verbleiben 24 Stunden bei 15°C und 100 % rel. Luftfeuchtigkeit in einer Inkubationskabine. Anschließend besprüht man die Pflanzen mit der Wirkstoffzubereitung taufeucht.

Nach Antrocknen der Spritzbrühe verbleiben die Pflanzen in einem Gewächshaus unter lichtdurchlässigen Inkubationshauben bei einer Temperatur von ca. 15°C und einer relativen Luftfeuchtigkeit von ca. 100%.

4 Tage nach der Inokulation erfolgt die Auswertung.

Bei diesem Test zeigen z.B. die Verbindungen der Herstellungsbeispiel (24), (30), (31), (34) und (43) bei einer beispielhaften Wirkstoffaufwandmenge von 250 g/ha einen Wirkungsgrad von 100 % im Vergleich zur unbehandelten Kontrolle.

### Beispiel L

### Pseudocercosporella herpotrichoides-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

Bei diesem Test zeigen z.B. die Verbindungen (15), (69) und (71) der Herstellungsbeispiele bei einer Wirkstoffaufwandmenge von 250 g/ha Wirkungsgrad von 100 % im Vergleich zur unbehandelten Kontrolle.

### Beispiel M

### Puccinia-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wäßrigen Agarlösung inokuliert. Nach Antrocknen besprüht man die Pflanzen mit der Wirkstoffzubereitung in der angegebenen Aufwandmenge. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

In diesem Test zeigen z.B. die Verbindungen der Herstellungsbeispiele(6) und (17) bei einer Wirkstoffaufwandmenge von 250 g/ha einen Wirkungsgrad von 100% im Vergleich zur unbehandelten Kontrolle.

### Beispiel N

### Pyricularia-Test (Reis) / protektiv

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit werden junge Reispflanzen mit der Wirkstoffzubereitung taufeucht bespritzt. 1 Tag nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Bei diesem Test zeigen z.B. die Verbindungen der Herstellungsbeispiele (2), (16), (17), (18), (19), (23), (24), (30), (32), (35), (41) und (48) bei einer Wirkstoffkonzentration von 0,05% einen Wirkungsgrad bis zu 100 % im Vergleich zur unbehandelten Kontrolle.

## Patentansprüche

1. Verbindungen der Formel (I) 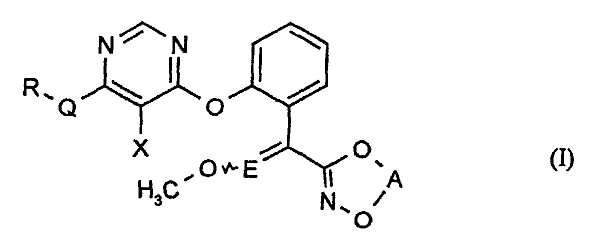 in welcher
A für gegebenenfalls durch Halogen substituiertes Alkandiyl mit 1 bis 5 Kohlenstoffatomen steht,
R für jeweils gegebenenfalls einfach bis zweifach durch Halogen, Alkyl, oder Hydroxy substiuiertes Cycloalkyl mit 3 bis 7 Kohlenstoffatomen;
für gegebenenfalls durch Alkyl mit 1 bis 4 Kohlenstoffatomen substituiertes Benzodioxanyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 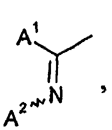 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl, Phenylalkyl, Phenylalkyloxy, Phenylalkylthio oder Heterocyclylalkyl mit jeweils 1 bis 3 Kohlenstoffatomen in den jeweiligen Alkylteilen,
E für -CH= oder Stickstoff steht,
Q für Sauerstoff, Schwefel, eine Einfachbindung oder für ein gegebenenfalls durch Methyl, Ethyl oder n- oder i-Propyl substituiertes Stickstoffatom steht und
X für Fluor, Chlor, Brom oder Iod steht.

2. Verbindungen der Formel (I) gemäß Anspruch 1, in welcher
A für jeweils gegebenenfalls durch Fluor oder Chlor substituiertes Methylen, Ethan-1,1-diyl, Ethan-1,2-diyl, Propan-1,1-diyl, Propan1,2-diyl, Propan-1,3-diyl, Butan-1,1-diyl, Butan-1,2-diyl, Butan-1,3-diyl oder Butan-2,2-diyl, steht
R für jeweils gegebenenfalls einfach bis zweifach durch Fluor, Chlor, Methyl, Ethyl oder Hydroxy substiuiertes Cyclopentyl oder Cyclohexyl;
für gegebenenfalls durch Methyl oder Ethyl substituiertes Benzodioxanyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 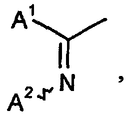 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
E für -CH= oder Stickstoff steht,
Q für Sauerstoff, Schwefel, eine Einfachbindung oder für ein gegebenenfalls durch Methyl substituiertes Stickstoffatom steht und
X für Fluor oder Chlor steht.

3. Schädlingsbekämpfungsmittel, **gekennzeichnet durch** einen Gehalt an mindestens einer Verbindung der Formel (I) nach Anspruch 1.

4. Verfahren zur Bekämpfung von Schädlingen, **dadurch gekennzeichnet, dass** man Verbindungen der Formel (I) nach Anspruch 1 auf Schädlinge und/oder ihren Lebensraum einwirken läßt.

5. Verwendung von Verbindungen der Formel (I) als Pflanzenschutzmittel nach den Ansprüchen 1 bis 3 zur Bekämpfung von unerwünschten Mikroorganismen.

6. Verfahren zur Herstellung von Pflanzenschutzmitteln, **dadurch gekennzeichnet, dass** man Verbindungen der Formel (I) nach den Ansprüchen 1 bis 3 mit Streckmitteln und/oder oberflächenaktiven Mitteln vermischt.

7. Verfahren zur Herstellung von Verbindungen der Formel (I) nach Anspruch 1, **dadurch gekennzeichnet, dass** man
a) Hydroxyverbindungen der allgemeinen Formel (II) 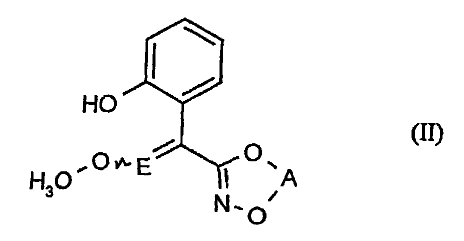 in welcher
A und E die in Anspruch 1 angegebenen Bedeutungen haben,
mit einem substituierten Halogenpyrimidin der allgemeinen Formel (III), 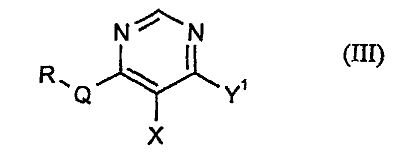 in welcher
R, Q und X die in Anspruch 1 angegebenen Bedeutungen haben und
Y¹ für Halogen steht,
gegebenenfalls in Gegenwart eines Verdünnungsmittels, gegebenenfalls in Gegenwart eines Säureakzeptors und gegebenenfalls in Gegenwart eines Katalysators, umsetzt, oder dass man
b) Phenoxypyrimidine der allgemeinen Formel (IV) 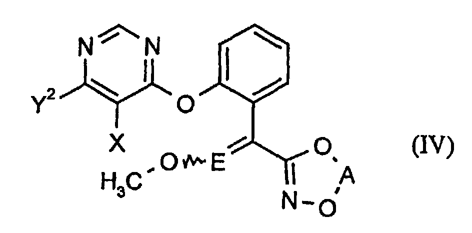 in welcher
A, E und X die in Anspruch 1 angegebenen Bedeutungen haben und
Y² für Halogen steht,
mit einer Ringverbindung der allgemeinen Formel (V),
R―Q―H (V)
in welcher
R und Q die in Anspruch 1 angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators, umsetzt.

8. Verbindungen der Formel (IV) 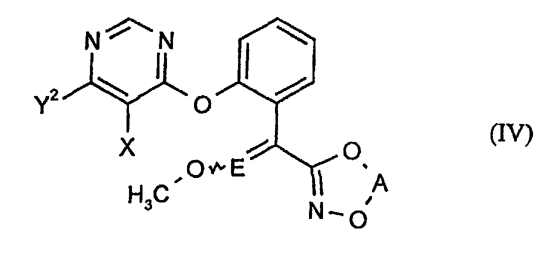 in welcher
A, E und X die Anspruch 1 angegebenen Bedeutungen haben und
Y² für Halogen steht.

9. Verbindung der Formel

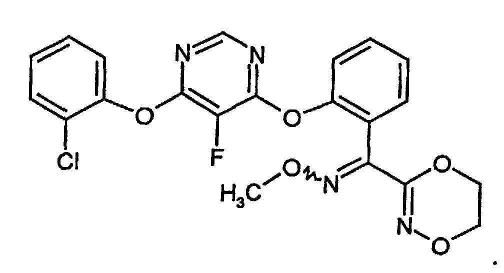

10. Verbindung der Formel

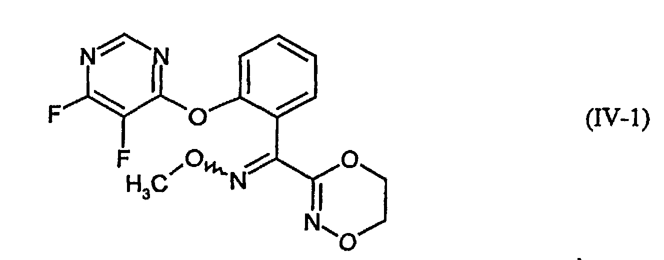

## Claims

1. Compounds of the formula (I) 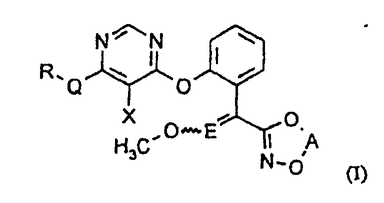 in which
A represents alkanediyl having 1 to 5 carbon atoms which is optionally substituted by halogen,
R represents cycloalkyl having 3 to 7 carbon atoms which is in each case optionally monosubstituted to disubstituted by halogen, alkyl or hydroxyl;
or represents benzodioxanyl which is optionally substituted by alkyl having 1 to 4 carbon atoms;
or represents phenyl or naphthyl, each of which is optionally monosubstituted to tetrasubstituted by identical or different substituents, other substituents which are possible preferably being selected from amongst the enumeration which follows:
halogen, cyano, nitro, amino, hydroxyl, formyl, carboxyl, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, alkylsulphinyl or alkylsulphonyl, each of which has 1 to 8 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy, each of which has 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylaminocarbonyl, dialkylaminocarbonyl, arylalkylaminocarbonyl, dialkylaminocarbonyloxy, alkenylcarbonyl or alkinylcarbonyl, each of which has 1 to 6 carbon atoms in the respective hydrocarbon chains;
cycloalkyl or cycloalkyloxy, each of which has 3 to 6 carbon atoms;
alkylene having 3 or 4 carbon atoms, oxyalkylene having 2 or 3 carbon atoms or dioxyalkylene having 1 or 2 carbon atoms, in each case optionally monosubstituted or tetrasubstituted by identical or different substituents from the series consisting of fluorine, chlorine, oxo, methyl, trifluoromethyl or ethyl;
or a group 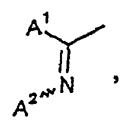 where
A¹ represents hydrogen, hydroxyl or alkyl having 1 to 4 carbon atoms or cycloalkyl having 1 to 6 carbon atoms and
A² represents hydroxyl, amino, methylamino, phenyl, benzyl, or represents alkyl or alkoxy having 1 to 4 carbon atoms, each of which is optionally substituted by cyano, hydroxyl, alkoxy, alkylthio, alkylamino, dialkylamino or phenyl, or represents alkenyloxy or alkinyloxy, each of which has 2 to 4 carbon atoms,
and also phenyl, phenoxy, phenylthio, benzoyl, benzoylethenyl, cinnamoyl, heterocyclyl, phenylalkyl, phenylalkyloxy, phenylalkylthio or heterocyclylalkyl, each of which has 1 to 3 carbon atoms in the respective alkyl moieties and each of which is optionally monosubstituted to trisubstituted in the cyclic moiety by halogen and/or straight-chain or branched alkyl or alkoxy having 1 to 4 carbon atoms,
E represents -CH= or nitrogen,
Q represents oxygen, sulphur, a single bond or a nitrogen atom which is optionally substituted by methyl, ethyl or n- or i-propyl and
X represents fluorine, chlorine, bromine or iodine.

2. Compounds of the formula (I) according to Claim 1 in which
A represents methylene, ethane-1,1-diyl, ethane-1,2-diyl, propane-1,1-diyl, propane-1,2-diyl, propane-1,3-diyl, butane-1,1-diyl, butane-1,2-diyl, butane-1,3-diyl or butane-2,2-diyl, each of which is optionally substituted by fluorine or chlorine,
R represents cyclopentyl or cyclohexyl, each of which is optionally monosubstituted to disubstituted by fluorine, chlorine, methyl, ethyl or hydroxyl;
or represents benzodioxanyl which is optionally substituted by methyl or ethyl;
or represents phenyl or naphthyl, each of which is optionally monosubstituted to tetrasubstituted by identical or different substituents, the substituents which are possible preferably being selected from amongst the enumeration which follows:
fluorine, chlorine, bromine, iodine, cyano, nitro, amino, hydroxyl, formyl, carboxyl, carbamoyl, thiocarbamoyl,
methyl, ethyl, n- or i-propyl, n-, i-, s- or t-butyl, 1-, 2-, 3-, neo-pentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimethylbutyl), 1-, .2-(2,3-dimethylbutyl), hydroxymethyl, hydroxyethyl, 3-oxobutyl, methoxymethyl, dimethoxymethyl,
methoxy, ethoxy, n- or i-propoxy,
methylthio, ethylthio, n- or i-propylthio, methylsulphinyl, ethylsulphinyl, methylsulphonyl or ethylsulphonyl,
vinyl, allyl, 2-methylallyl, propen-1-yl, crotonyl, propargyl, vinyloxy, allyloxy, 2-methylallyloxy, propen-1-yloxy, crotonyloxy, propargyloxy;
trifluoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
methylamino, ethylamino, n- or i-propylamino, dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
in each case divalent propanediyl, ethyleneoxy, methylenedioxy, ethylenedioxy, each of which is optionally monosubstituted to tetrasubstituted by identical or different substituents from the series consisting of fluorine, chlorine, oxo, methyl or trifluoromethyl,
or a group 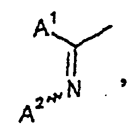 where
A¹ represents hydrogen, methyl or hydroxyl and
A² represents hydroxyl, methoxy, ethoxy, amino, methylamino, phenyl, benzyl or hydroxyethyl, and
phenyl, phenoxy, phenylthio, benzoyl, benzoylethenyl, cinnamoyl, benzyl, phenylethyl, phenylpropyl, benzyloxy, benzylthio, 5,6-dihydro-1,4,2-dioxazin-3-ylmethyl, triazolylmethyl, benzoxazol-2-ylmethyl, 1,3-dioxan-2-yl, benzimidazol-2-yl, dioxol-2-yl, oxadiazolyl, each of which is optionally monosubstituted to trisubstituted in the cyclic moiety by halogen and/or straight-chain or branched alkyl or alkoxy having 1 to 4 carbon atoms,
E represents -CH= or nitrogen,
Q represents oxygen, sulphur, a single bond or a nitrogen atom which is optionally substituted by methyl, and
X represents fluorine or chlorine.

3. Pesticides, **characterized in that** they comprise at least one compound of the formula (I) according to Claim 1.

4. Method of controlling pests, **characterized in that** compounds of the formula (I) according to Claim 1 are allowed to act on pests and/or their environment.

5. Use of compounds of the formula (I) as crop protection agents according to Claims 1 to 3 for controlling undesirable microorganisms.

6. Process for the preparation of crop protection agents, **characterized in that** compounds of the formula (I) according to Claims 1 to 3 are mixed with extenders and/or surfactants.

7. Process for the preparation of compounds of the formula (I) according to Claim 1, **characterized in that**
a) hydroxy compounds of the general formula (II) 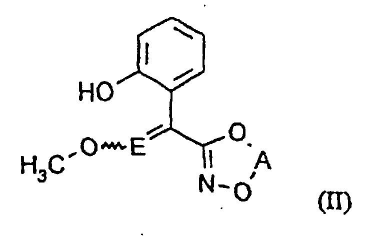 in which
A and E have the meanings given in Claim 1,
are reacted with a substituted halogenopyrimidine of the general formula (III) 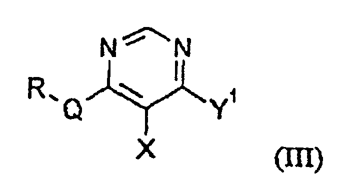 in which
R, Q and X have the meanings given in Claim 1 and
Y¹ represents halogen,
if appropriate in the presence of a diluent, if appropriate in the presence of an acid acceptor and if appropriate in the presence of a catalyst, or **in that**
b) phenoxypyrimidines of the general formula (IV) 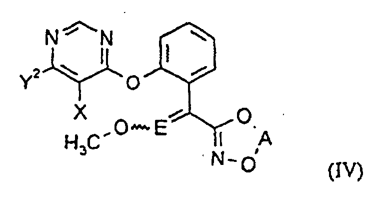 in which
A, E and X have the meanings given in Claim 1 and
Y² represents halogen
are reacted with a cyclic compound of the general formula (V)
R―Q―H (V)
in which
R and Q have the meanings given in Claim 1,
if appropriate in the presence of a diluent, if appropriate in the presence of an acid acceptor and if appropriate in the presence of a catalyst.

8. Compounds of the formula (IV) 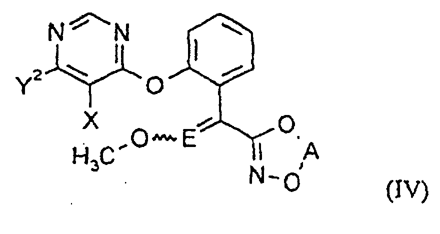 in which
A, E and X have the meanings given in Claim 1 and
Y² represents halogen.

9. Compound of the formula

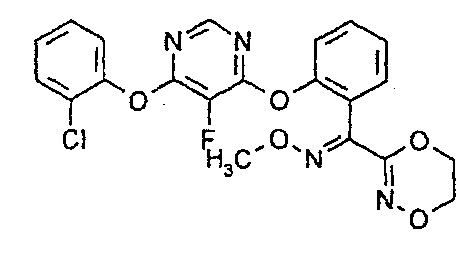

10. Compound of the formula

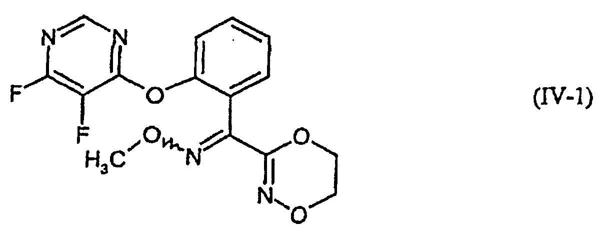

## Revendications

1. Composés de formule (I) 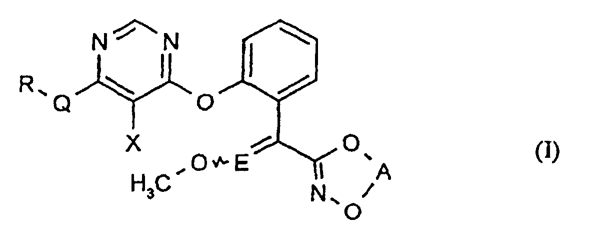 dans laquelle
A représente un groupe alcanediyle ayant 1 à 5 atomes de carbone, éventuellement substitué par un halogène,
R représente un groupe cycloalkyle ayant 3 à 7 atomes de carbone, éventuellement substitué dans chaque cas une
ou deux fois par un halogène, un radical alkyle ou hydroxy,
un groupe benzodioxannyle éventuellement substitué par un radical alkyle ayant 1 à 4 atomes de carbone,
ou bien
un groupe phényle ou naphtyle éventuellement substitué dans chaque cas une à quatre fois identiques ou différentes, les substituants possibles étant choisis de préférence parmi ceux qui sont énumérés ci-après :
halogène, cyano, nitro, amino, hydroxy, formyle, carboxy, carbamoyle, thiocarbamoyle ;
alkyle, hydroxyalkyle, oxo-alkyle, alkoxy, alkoxyalkyle, alkylthioalkyle, dialkoxyalkyle, alkylthio, alkylsulfinyle ou aklylsulfonyle étant chacun linéaire ou ramifié et ayant chacun 1 à 8 atomes de carbone ;
alcényle ou alcényloxy étant chacun linéaire ou ramifié et ayant chacun 2 à 6 atomes de carbone ;
halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ;
halogénalcényle ou halogénalcényloxy étant chacun linéaire ou ramifié et ayant chacun 2 à 6 atomes de carbone et 1 à 11 atomes d'halogènes identiques ou différents ;
alkylamino, dialkylamino, alkylcarbonyle, alkylcarbonyloxy, alkoxycarbonyle, alkylaminocarbonyle, dialkylaminocarbonyle, arylalkylaminocarbonyle, dialkylaminocarbonyloxy, alcénylcarbonyle ou alcynylcarbonyle étant chacun linéaire ou ramifié et ayant chacun 1 à 6 atomes de carbone dans les chaînes hydrocarbonées respectives ;
cycloalkyle ou cycloalkyloxy ayant chacun 3 à 6 atomes de carbone ;
alkylène ayant 3 ou 4 atomes de carbone, oxyalkylène ayant 2 ou 3 atomes de carbone ou dioxyalkylène ayant 1 ou 2 atomes de carbone, chacun ayant deux liaisons et portant éventuellement un à quatre substituants fluor, chlore, oxo, méthyle, trifluorométhyle ou éthyle identiques ou différents ;
ou un groupement 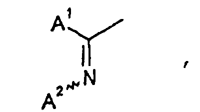 dans lequel
A¹ représente l'hydrogène, un groupe hydroxy ou un groupe alkyle ayant 1 à 4 atomes de carbone ou cycloalkyle ayant 1 à 6 atomes de carbone et
A² représente un groupe hydroxy, amino, méthylamino, phényle, benzyle ou un groupe alkyle ou alkoxy ayant 1 à 4 atomes de carbone et chacun étant substitué, le cas échéant, par un radical cyano, hydroxy, alkoxy, alkylthio, alkylamino, dialkylamino ou phényle, ou un groupe alcényloxy ou alcynyloxy ayant chacun 2 à 4 atomes de carbone,
de même qu'un groupe phényle, phénoxy, phénylthio, benzoyle, benzoyléthényle, cynnamoyle, hétérocyclyle, phénylalkyle, phénylalkyloxy, phénylalkylthio ou hétérocyclylalkyle ayant dans chaque cas 1 à 3 atomes de carbone dans les parties alkyle respectives et chacun étant éventuellement substitué dans le noyau une à trois fois par un halogène et/ou un radical alkyle ou alkoxy linéaire ou ramifié ayant 1 à 4 atomes de carbone,
E représente un groupe -CH= ou l'azote,
Q représente l'oxygène, le soufre, une liaison simple ou un atome d'azote éventuellement substitué par un radical méthyle, éthyle, n-propyle ou isopropyle et
X représente le fluor, le chlore, le brome ou l'iode.

2. Composés de formule (I) suivant la revendication 1, dans laquelle
A représente un groupe méthylène, éthane-1,1-diyle, éthane-1,2-diyle, propane-1,1-diyle, propane-1,2-diyle, propane-1,3-diyle, butane-1,1-diyle, butane-1,2-diyle, butane-1,3-diyle ou butane-2,2-diyle, chacun étant éventuellement substitué par du fluor ou du chlore,
R représente un groupe cyclopentyle ou cyclohexyle portant chacun, le cas échéant, un ou deux substituants fluor, chlore, méthyle, éthyle ou hydroxy identiques ou différents ;
un groupe benzodioxannyle éventuellement substitué par un radical méthyle ou éthyle ;
ou bien un groupe phényle ou naphtyle portant chacun, le cas échéant, un à quatre substituants identiques ou différents, les substituants possibles étant choisis de préférence parmi ceux qui sont énumérés ci-après :
fluor, chlore, brome, iode, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, 1-, 2-, 3-néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-, 2-(2,3-diméthylbutyle), hydroxyméthyle, hydroxyéthyle, 3-oxobutyle, méthoxyméthyle, diméthoxyméthyle,
méthoxy, éthoxy, n-propoxy ou isopropoxy,
méthylthio, éthylthio, n-propylthio, isopropylthio, méthylsulfinyle, éthylsulfinyle, méthylsulfonyle ou éthylsulfonyle,
vinyle, allyle, 2-méthylallyle, propén-1-yle, crotonyle, propargyle, vinyloxy, allyloxy, 2-méthylallyloxy, propén-1-yloxy, crotonyloxy, propargyloxy ; trifluorométhyle, trifluoréthyle,
difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle,
méthylamino, éthylamino, n-propylamino, isopropylamino, diméthylamino, diéthylamino,
acétyle, propionyle, méthoxycarbonyle, éthoxycarbonyle, méthylaminocarbonyle, éthylaminocarbonyle, diméthylaminocarbonyle, diéthylaminocarbonyle, diméthylaminocarbonyloxy, diéthylaminocarbonyloxy, benzylaminocarbonyle, acryloyle, propioloyle, cyclopentyle, cyclohexyle,
propanediyle, éthylèneoxy, méthylènedioxy, éthylènedioxy ayant chacun deux liaisons et portant chacun, le cas échéant, un à quatre substituants fluor, chlore, oxo, méthyle ou trifluorométhyle identiques ou différents
ou un groupement 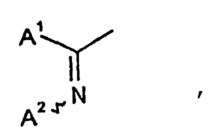 dans lequel
A¹ représente l'hydrogène, un groupe méthyle ou hydroxy et
A² est un groupe hydroxy, méthoxy, éthoxy, amino, méthylamino, phényle, benzyle ou hydroxyéthyle,
ainsi que phényle, phénoxy, phénylthio, benzoyle, benzoyléthényle, cinnamoyle, benzyle, phényléthyle, phénylpropyle, benzyloxy, benzylthio, 5,6-dihydro-1,4,2-dioxazine-3-ylméthyle, triazolylméthyle, benzoxazole-2-ylméthyle, 1,4-dioxanne-2-yle, benzimidazole-2-yle, dioxole-2-yle, oxadiazolyle portant chacun, le cas échéant dans la partie cyclique, un à trois substituants halogène et/ou alkyle ou alkoxy linéaire ou ramifié ayant 1 à 4 atomes de carbone,
E représente un groupe -CH= ou l'azote,
Q est l'oxygène, le soufre, une liaison simple ou un atome d'azote éventuellement substitué par un radical méthyle et
X représente le fluor ou le chlore.

3. Compositions pesticides, **caractérisées par** une teneur en au moins un composé de formule (I) suivant la revendication 1.

4. Procédé de lutte contre des parasites, **caractérisé en ce qu'**on fait agir des composés de formule (I) suivant la revendication 1 sur les parasites et/ou sur leur milieu.

5. Utilisation de composés de formule (I) suivant les revendications 1 à 3 comme agents phytosanitaires pour la lutte contre des micro-organismes indésirables.

6. Procédé de préparation de compositions phytosanitaires, **caractérisé en ce qu'**on mélange des composés de formule (I) suivant les revendications 1 à 3 avec des diluants et/ou des agents tensio-actifs.

7. Procédé de production de composés de formule (I) suivant la revendication 1, **caractérisé en ce que** :
a) on fait réagir des composés hydroxylés de formule générale (II) 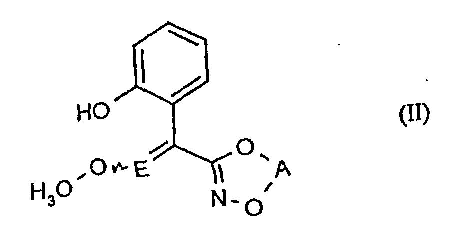 dans laquelle
A et E ont les définitions indiquées dans la revendication 1,
avec une halogénopyrimidine substituée de formule générale (III) 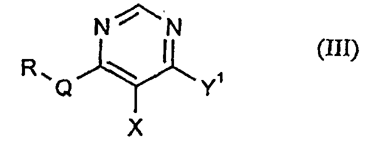 dans laquelle
R, Q et X ont les définitions indiquées dans la revendication 1 et
Y¹ représente un halogène,
éventuellement en présence d'un diluant, le cas échéant en présence d'un accepteur d'acide et en présence éventuelle d'un catalyseur, ou **en ce que** :
b) on fait réagir des phénoxypyrimidines de formule générale (IV) 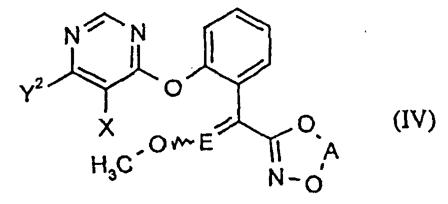 dans laquelle
A, E et X ont les définitions indiquées dans la revendication 1 et
Y² représente un halogène,
avec un composé cyclique de formule générale (V)
R―Q―H (V)
dans laquelle
R et Q ont les définitions indiquées dans la revendication 1,
le cas échéant en présence d'un diluant, éventuellement en présence d'un accepteur d'acide et en présence d'un catalyseur.

8. Composés de formule (IV) 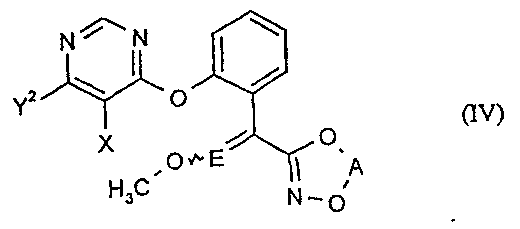 dans laquelle
A, E et X ont les définitions indiquées dans la revendication 1 et
Y² représente un halogène.

9. Composé de formule :

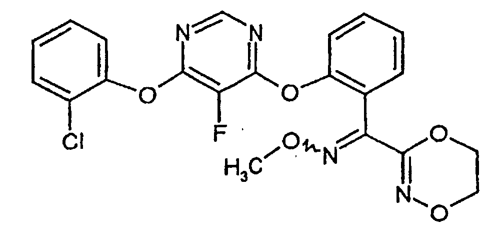

10. Composé de formule :
